# FASCICULE DE BREVET EUROPEEN

(11) **EP 3 638 659 B1**
(45) Date de publication et mention de la délivrance du brevet: **15.12.2021**
(21) Numéro de dépôt: 18737337.8
(22) Date de dépôt: 11.06.2018
(51) Int. Cl.: C07D 401/14, C07F 9/6558, C07F 5/00, C09K 11/06, G01N 33/58

(54) **NOUVEAUX AGENTS MONO ET DI-ANTENNES COMPLEXANTS HYDROSOLUBLES ET COMPLEXES DE LANTHANIDE CORRESPONDANTS**
NEUE WASSERLÖSLICHE MONOVERZWEIGTE UND DIVERZWEIGTE KOMPLEXBILDNER UND ENTSPRECHENDE LANTHANIDKOMPLEXE
NEW WATER SOLUBLE MONO-BRANCHED AND DI-BRANCHED COMPLEXING AGENTS, AND CORRESPONDING LANTHANIDE COMPLEXES

(30) Priorité: 12.06.2017 FR 1755214
(43) Date de publication de la demande: 22.04.2020
(73) Titulaire: CISBIO BIOASSAYS, 30200 Codolet (FR)
(72) Inventeur: LAMARQUE, Laurent, 30290 Saint-Victor La Coste (FR); ZWIER, Jurriaan, 30650 Rochefort-du-Gard (FR); BOURRIER, Emmanuel, 30200 Bagnols-sur-Ceze (FR)
(74) Mandataire: Cabinet Beau de Loménie
(86) Numéro de dépôt international: PCT/FR2018/051354
(87) Numéro de publication internationale: WO 2018/229408

(56) Documents cités:
- WO-A1-2013/011236
- WO-A1-2014/147288
- WO-A1-2014/162105
- WO-A1-2016/066641
- MATTHIEU STARCK ET AL: "Structural Control of Cell Permeability with Highly Emissive Europium(III) Complexes Permits Different Microscopy Applications", CHEMISTRY - A EUROPEAN JOURNAL, vol. 22, no. 2, 11 janvier 2016 (2016-01-11), pages 570-580, XP055434704, ISSN: 0947-6539, DOI: 10.1002/chem.201504103

## Description

La présente invention a pour objet des agents complexants ou ligands hydrosolubles, des complexes de lanthanide obtenus à partir de ces agents complexants, et l'utilisation de ces complexes de lanthanide pour marquer des molécules et les détecter par des techniques de fluorescence en temps résolu.

### Etat de la technique

Les complexes de lanthanide ont vu leur utilisation augmenter de manière très importante depuis une vingtaine d'années dans le domaine des sciences de la vie. Ces composés fluorescents présentent en effet des caractéristiques spectroscopiques intéressantes, qui en font des marqueurs de choix pour détecter des molécules biologiques. Ces composés fluorescents sont particulièrement appropriés pour être utilisés en conjonction avec des fluorophores compatibles pour effectuer des mesures de FRET (acronyme de l'expression anglaise « Förster Resonnance Energy Transfer »), dont l'application pour étudier les interactions entre biomolécules est exploitée de manière commerciale par plusieurs sociétés, dont Cisbio Bioassays et sa gamme de produits HTRF^{®}. La durée de vie relativement longue des complexes de lanthanides permet également d'effectuer des mesures de fluorescence en temps résolu, c'est-à-dire avec un délai après excitation des fluorophores, ce qui permet de limiter les interférences de fluorescence dues au milieu de mesure. Cette dernière caractéristique est d'autant plus utile que le milieu de mesure se rapproche d'un milieu biologique, qui comprend de nombreuses protéines dont la fluorescence pourrait interférer avec celle des composés étudiés.

De nombreux complexes de lanthanide ont été décrits. Latva et al par exemple, ont divulgué 41 complexes d'Eu(III) et de Tb(III), dont ils ont étudié la luminescence (Journal of Luminescence 1997, 75, 149). Le composé **39** en particulier, est constitué d'un cycle 1,4,7-triazacyclononane (ci-après « TACN »), dont les atomes d'azote sont substitués par des chromophores dérivés de phényléthynylpyridine. Bien que le rendement quantique du complexe constitué de ce chromophore et d'Eu(III) soit considéré comme bon par les auteurs, ce complexe n'est pas adapté au couplage avec une biomolécule. Par ailleurs, l'utilisation de ce composé en milieu aqueux peut être problématique puisqu'il est très hydrophobe. Enfin, l'absorption de ce complexe est optimale à 315 nm, alors que la longueur d'onde d'excitation souvent utilisée dans les lecteurs de plaques pour les dosages biologiques est plutôt à 337 nm.

D'Aléo et al ont décrit la synthèse de complexes de lanthanide constitués de trois ligands dérivés de l'acide dipicolonique (Inorganic Chemistry 2008, 47, 10258). L'un de ces ligands (L1) est constitué d'une molécule d'acide dipicolinique substituée par un groupe phényléthynyl, lui-même portant un éther-oxyde de polyéthylèneglycol (ci-après « PEG ») sur le groupe phényle. D'après les auteurs, le groupe PEG confère à ce produit une bonne solubilité en milieux aqueux et dans les solvants organiques. Toutefois, ces complexes ne sont pas suffisamment stables en milieu aqueux et ne sont pas utilisables dans une réaction de bioconjuguaison.

Plusieurs autres complexes de lanthanide ont été divulgués et certains sont exploités de manière commerciale : on peut citer en particulier les cryptates macropolycycliques de lanthanide (EP-A-0 180 492, EP-A-0 321 353, EP-A-0 601 113, WO 2001/96877, WO 2008/063721), les complexes de lanthanide comportant un motif dérivé de la coumarine lié à un motif diéthylènetriamine penta-acide (US 5,622,821), et ceux comprenant des dérivés de pyridine (US 4,920,195, US 4,761,481), de bipyridine (US 5,216,134), ou de terpyridine (US 4,859,777, US 5,202,423, US 5,324,825).

La demande de brevet WO 2013/011236 décrit des agents complexants de formule :

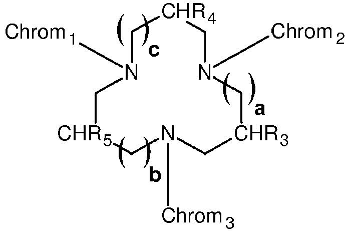

Dans cette demande les inventeurs envisagent des complexes comportant trois chromophores afin d'augmenter la brillance. De plus pour rendre hydrosoluble ces complexes, les inventeurs utilisent des groupements PEG. Bien que ces groupements PEG soient neutres d'un point de vue « charge globale », ils confèrent aux complexes des propriétés d'adsorption sur le plastique et le verre ce qui rend peu aisée leur utilisation dans un dosage immunologique.

La demande de brevet WO 2014/111661 décrit des agents complexants, comprenant trois groupes chromophores, représentés par la formule :

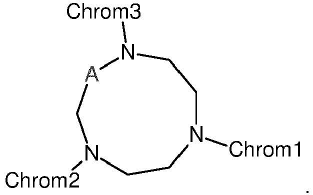

Dans cette demande, les inventeurs ont remplacé les groupements PEG par des groupements chargés : anioniques ou cationiques. Ces complexes sont parfaitement solubles et ne s'adsorbent plus sur le plastique ou le verre. Ces complexes présentent des structures en étoile assez volumineuses et possèdent des propriétés de brillance très élevées.

La demande de brevet WO 2014/162105 concerne des complexes de lanthanide comprenant au moins deux groupes bétaïne sur la partie organique leur conférant des propriétés intéressantes en terme de solubilité dans l'eau et les milieux biologiques. Ces groupes solubilisants sont présentés comme permettant de limiter les phénomènes d'adsorption non spécifique avec les cellules vivantes. En revanche ces complexes possèdent trois chromophores identiques ou différents ce qui comme dans la demande WO 2014/111661 génère des structures en étoile assez volumineuses et de bonnes propriétés de brillance.

Les demandes de brevet WO 2014/147288 et WO 2016/066641 décrivent des complexes de TACN possédant des coefficients d'absorption molaires élevés. Ces complexes présentent une brillance elévée mais possèdent également trois chromophores. Les inventeurs cherchent les complexes les plus brillants possibles afin d'avoir un transfert d'énergie optimal (FRET). Les structures en étoile des complexes est encore une fois volumineuses et leur brillance est importante.

L'introduction de trois chromophores sur un macrocycle de type triazacyclononane n'est pas toujours un avantage puisque les chromophores sont des synthons provenant d'une synthèse longue et fastidieuse. La présence de trois chromophores dans un complexe de TACN augmente la taille de la molécule ce qui augmente les emcombrements stériques avec les biomolécules. Néanmoins, plus le nombre de chromophores est important plus la brillance est élevée, puisque ce paramètre dépend du rendement quantique et du coefficient d'absorption molaire (epsilon) de la molécule. En introduisant trois chromophores sur une molécule, la brillance est augmentée.

Dans la présente invention nous avons découvert qu'il n'y a pas de corrélation entre la brillance d'un complexe et sa capacité à détecter une cible biologique dans un immunoessai basé sur du FRET en temps résolu dans lequel le complexe est utilisé comme donneur d'énergie. Cette invention vise à palier aux incovénients de l'art antérieur, c'est-à-dire de simplifier la synthèse en introduisant un ou deux chromophores, de réduire les encombrements stériques et finalement de disposer d'une sonde fluorescente ayant au moins les mêmes performances en terme de transfert d'énergie.

La présente invention vise donc à fournir des complexes de lanthanide fluorescents présentant une brillance inférieure à celle des composés de l'art antérieur lorsqu'ils sont excités aux alentours de 337 nm mais dont la capacité à détecter une cible biologique dans un immunoessai basé sur du FRET en temps résolu est comparable voire meilleure que celle des analogues portant trois chromophores. Ces complexes sont d'autre part dotés d'une bonne solubilité en milieu aqueux, d'un spectre d'émission adapté à leur utilisation dans des expériences de FRET, ainsi que d'une bonne praticité pour le marquage de biomolécules puisqu'ils sont de plus petite taille.

### Brève description des figures

Les figures 1 à 3 représentent respectivement le spectre UV, le chromatogramme et le spectre de masse d'un complexe représentatif de l'invention.
La figure 4 représente schématiquement un immunoessai mis en œuvre pour tester l'efficacité d'un complexe représentatif de l'invention.
La figure 5 représente le signal de FRET (Delta F) mesuré lors de la mise en œuvre de l'immunoessai de la figure 4.
La figure 6 représente schématiquement un immunoessai mis en œuvre pour tester l'efficacité d'un complexe représentatif de l'invention.
La figure 7 représente représente le signal de FRET (Delta F) mesuré lors de la mise en œuvre de l'immunoessai de la figure 6.

### AGENTS COMPLEXANTS

Les agents complexants selon l'invention sont les composés de formule (I) : 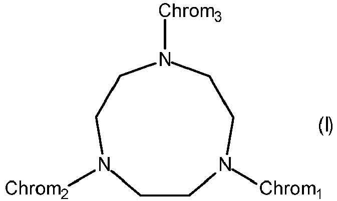 dans laquelle :
- Chrom₁, Chrom₂ et Chrom₃ représentent indépendamment l'un de l'autre un groupe de formule : 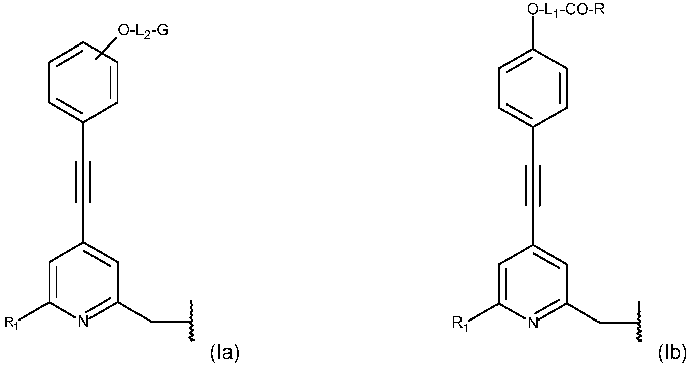 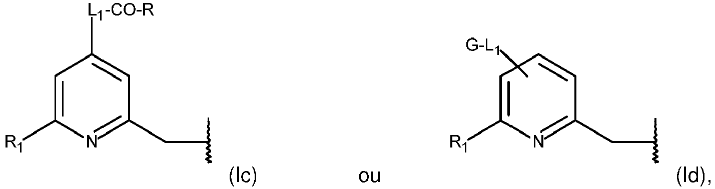 étant entendu que le composé de formule (I) comprend (nécessairement) (i) un ou deux groupes choisis parmi les groupes (la) et (Ib) et (ii) au moins un groupe L₁-CO-R ;
- R est un groupe ―OR₂ ou -NH-E ;
- R₁ est un groupe ―CO₂H ou ―P(O)(OH)R₃ ;
- R₂ est H ou un (C₁-C₄)alkyle ;
- R₃ est un (C₁-C₄)alkyle, de préférence un méthyle ; un phényle éventuellement substitué par un groupe ―SO₃⁻, ce dernier étant de préférence en position méta ou para ; ou un benzyle ;
- L₁ est une liaison directe ; un groupe ―(CH₂)ᵣ - éventuellement interrompu par au moins un atome choisi parmi un atome d'oxygène, un atome d'azote et un atome de soufre ; un groupe -CH=CH- ; un groupe ―CH=CH-CH₂- ; un groupe ―CH₂-CH=CH- ; ou un groupe PEG ;
- L₂ est un groupe de liaison divalent ;
- G est un groupe réactif choisi parmi une amine éventuellement protégée sous forme -NHBoc, un ester de succinimidyle, un haloacétamide, une hydrazine, un isothiocyanate, un groupe maléimide, ou un acide carboxylique éventuellement protégé sous la forme d'un groupe ―CO₂Me ou -CO₂tBu ;
- E est un groupe ―CH₂-(CH₂)ₛ-CH₂-SO₃⁻ ou ―(CH₂)ₛ-N⁺Alk₁Alk₂Alk₃, ou une sulfobétaïne, ladite sulfobétaïne ayant l'une des formules ci-après : 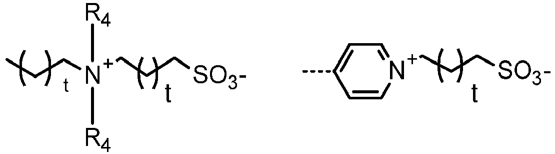 avec R₄ qui représente un (C₁-C₆)alkyle, et t qui est égal à 1, 2, 3, 4, 5 ou 6 ;
- r est un entier allant de 1 à 6, de préférence de 1 à 3 ;
- s est 0, 1 ou 2 ;
- Alk₁, Alk₂, Alk₃, qui peuvent être identiques ou différents, représentent un (C₁-C₆)alkyle.

Les agents complexants selon l'invention comprennent donc soit un soit deux groupes choisis parmi les groupes (la) et (Ib) et, par voie de conséquence, soit un soit deux groupes choisis parmi les groupes (Ic) et (Id) - puisque la structure de formule (I) comprend trois chromophores (Chrom₁, Chrom₂ et Chrom₃). Une condition supplémentaire pour les composés de formule (I) est qu'au moins un groupe L₁-CO-R doit être présent dans la structure.

Par groupe PEG on entend un groupe polyéthylène glycol de formule -CH₂―(CH₂OCH₂)_{y}-CH₂OCH₃, y étant un nombre entier allant de 1 à 5.

La sulfobétaïne est un groupe choisi parmi : 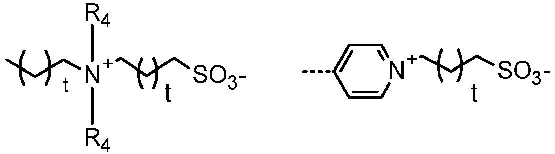 avec R₄ qui représente un (C₁-C₆)alkyle, de préférence un méthyle ou éthyle, et t qui est égal à 1, 2, 3, 4, 5 ou 6, et de préférence qui est égal à 1 ou 2, la sulfobétaïne de formule ―(CH₂)₂N⁺(CH₃)₂-(CH₂)₃-SO₃⁻ étant préférée. En fonction du pH, les groupes -SO₃H, -CO₂H et -PO(OH)₂ sont sous forme déprotonée ou pas. Ces groupes désignent donc dans la suite du texte également les groupes -SO₃⁻, -CO₂⁻ et -PO(OH)O⁻, et vice-versa.

Une première famille préférée d'agents complexants est constituée des composés de formule (I) où Chrom₁ est un groupe de formule (la) et Chrom₂ et Chrom₃ sont chacun un groupe de formule (Ic), identique ou différent. Dans un mode de réalisation, Chrom₂ et Chrom₃ sont identiques. Dans un autre mode de réalisation, qui peut être combiné au mode de réalisation précédent, R₁ est un groupe - CO₂H ou -P(O)(OH)R₃ dans lequel R₃ est un (C₁-C₄)alkyle ou un phényle.

Une deuxième famille préférée d'agents complexants est constituée des composés de formule (I) où Chrom₁ et Chrom₂ sont chacun un groupe de formule (Ib), identique ou différent, et Chrom₃ est un groupe de formule (Id). Dans un mode de réalisation, Chrom₁ et Chrom₂ sont identiques. Dans un autre mode de réalisation, qui peut être combiné au mode de réalisation précédent, R₁ est un groupe ―CO₂H ou -P(O)(OH)R₃ dans lequel R₃ est un (C₁-C₄)alkyle ou un phényle.

Parmi ces deux familles préférées, des sous-familles préférées sont celles où les agents complexants comprennent une ou plusieurs des caractéristiques ci-après :
- R₂ est H ;
- L₁ est est une liaison directe ; un groupe ―(CH₂)ᵣ― éventuellement interrompu par au moins un atome choisi parmi un atome d'oxygène et un atome de soufre, et r = 2 ou 3 ; un groupe - CH=CH- ; un groupe ―CH=CH-CH₂― ; ou un groupe ―CH₂-CH=CH- ;
- E est un groupe ―CH₂-(CH₂)ₛ-CH₂-SO₃⁻ avec s = 0 ou 1 ; ―(CH₂)ₛ-N⁺Alk₁Alk₂Alk₃ avec Alk₁, Alk₂ Alk₃, identiques ou différents, représentant un (C₁-C₄)alkyle et s = 0 ou 1 ; ou un groupe de formule : 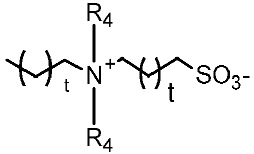
dans laquelle R₄ est un (C₁-C₄)alkyle et t est 1 ou 2.

Dans un mode de réalisation de l'invention, lorsque les agents complexants de formule (I) comprennent plusieurs groupes E, au plus l'un de ces groupes représente une sulfobétaïne.

Le groupe réactif G porté par un bras d'espacement L₁ ou L₂, permet de coupler les composés selon l'invention avec une espèce que l'on souhaite rendre fluorescente, par exemple une molécule organique, un peptide ou une protéine. Les techniques de conjugaison de deux molécules organiques sont basées sur l'utilisation de groupes réactifs et relèvent des connaissances générales de l'homme du métier. Ces techniques classiques sont décrites par exemple dans Bioconjugate Techniques, G.T. Hermanson, Academic Press, Second Edition 2008, p. 169-211.

Typiquement, le groupe réactif est un groupe électrophile ou nucléophile qui peut former une liaison covalente lorsqu'il est respectivement mis en présence d'un groupe nucléophile ou électrophile approprié. La réaction de conjugaison entre un composé selon l'invention comportant un groupe réactif et une molécule organique, un peptide ou une protéine portant un groupe fonctionnel entraîne la formation d'une liaison covalente comportant un ou plusieurs atomes du groupe réactif.

Le groupe réactif G est une amine (éventuellement protégée sous forme -NHBoc), un ester de succinimidyle, un haloacétamide, une hydrazine, un isothiocyanate, un groupe maléimide, ou un acide carboxylique (éventuellement protégé sous la forme d'un groupe -CO₂Me, -CO₂tBu). Dans ce dernier cas, l'acide devra être activé sous forme d'ester pour pouvoir réagir avec une espèce nucléophile.

Le groupe réactif G est lié à l'agent complexant via un bras d'espacement L₁ ou L₂, constitué de manière avantageuse par un radical organique bivalent. En particulier, le bras d'espacement L₂ peut être choisi parmi :
- une liaison directe;
- un groupe alkylène linéaire ou ramifié en C₁-C₂₀, de préférence en C₁-C₈, contenant éventuellement une ou plusieurs doubles ou triples liaisons ;
- un groupe cycloalkylène en C₅-C₈ ; un groupe arylène en C₆-C₁₄;
   lesdits groupes alkylène, cycloalkylène ou arylène contenant éventuellement un ou plusieurs hétéroatomes, tels que l'oxygène, l'azote, le soufre, le phosphore ou un ou plusieurs groupe(s) carbamoyle ou carboxamido, et lesdits groupes alkylène, cycloalkylène ou arylène étant éventuellement substitués par 1 à 5, de préférence 1 à 3, groupes alkyle en C₁-C₈, aryle en C₆-C₁₄, sulfonate ou oxo.
- un groupe choisi parmi les groupes divalents de formules suivantes : —(CH2)ₙ— _{;}—(CH₂)ₙ—O—(CH₂)ₘ—O—(CH₂)ₚ— ; 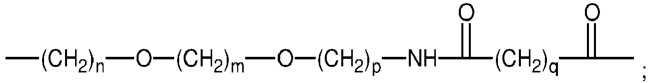 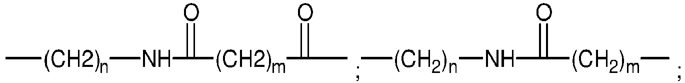 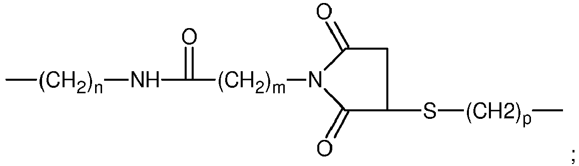 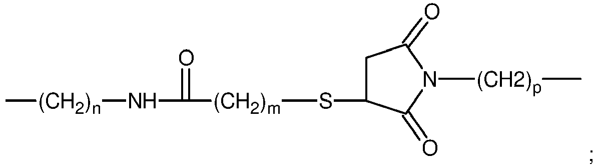 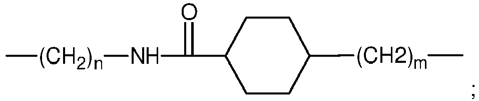 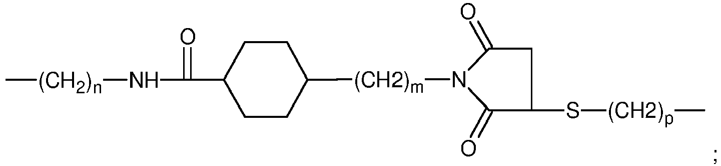 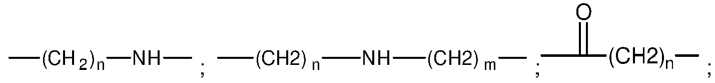 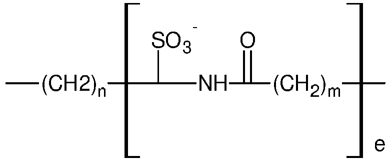 dans lesquelles n, m, p, q sont des nombres entiers de 1 à 16, de préférence de 1 à 5 et e est un nombre entier allant de 1 à 6, de préférence de 1 à 4.

Dans les groupes de formule (la), le groupe ―L₂-G est de préférence constitué d'un groupement réactif G tel que défini ci-dessus, et d'un bras d'espacement L₂ constitué d'une chaîne alkylène comprenant de 1 à 5 atomes de carbone ou d'un groupe choisi parmi les groupes de formule : 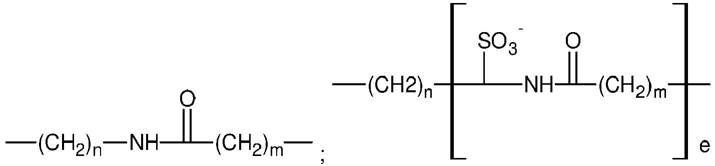 où n, m, sont des nombres entiers de 1 à 16, de préférence de 1 à 5 et e est un nombre entier allant de 1 à 6, de préférence de 1 à 4, le groupe G étant lié à l'une ou l'autre extrémité de ces groupes divalents.

### COMPLEXES

L'invention concerne également les complexes de lanthanide constitués d'un atome de lanthanide complexé par un agent complexant tel que décrit ci-dessus, le lanthanide étant choisi parmi : Eu³⁺, Sm³⁺, Tb³⁺, Gd³⁺, Dy³⁺, Nd³⁺, Er³⁺. De préférence, le lanthanide est Tb³⁺ ou Eu³⁺ et de manière encore plus préférée Eu³⁺.

Ces complexes sont préparés en faisant réagir les agents complexants selon l'invention avec un sel de lanthanide. Ainsi la réaction entre un équivalent d'agent complexant et 1 à 5 équivalents de sel de lanthanide (europium ou terbium sous forme de chlorures, d'acétates ou de triflates) dans un solvant (acétonitrile, méthanol ou autre solvant compatible avec ces sels) ou un tampon conduit au complexe correspondant après quelques minutes d'agitation.

Comme indiqué précédemment, les complexes fluorescents obtenus présentent d'excellentes propriétés photophysiques, en particulier en ce qui concerne leur rendement quantique, la durée de vie de leur luminescence et leur spectre d'excitation qui est très bien adapté à une excitation laser à environ 337 nm ou lampe flash. De plus la répartition des bandes de leurs spectres d'émission est centrée autour de 620 nm conférant ainsi aux complexes des propriétés exceptionnelles et très favorables dans une utilisation de FRET avec des accepteurs de type cyanine ou allophycocyanine (telle que la XL665 commercialisée par Cisbio Bioassays). Du fait de la grande stabilité de ces complexes dans les milieux biologiques contenant la plupart des cations divalents (Ca²⁺, Mg²⁺...) ou de l'EDTA, leur luminescence reste excellente comparée aux complexes portant trois chromophores de l'art antérieur.

### CONJUGUES

Les agents complexants et complexes de lanthanide selon l'invention comportant un groupe G, sont particulièrement adaptés au marquage de molécules organiques ou biologiques comportant un groupe fonctionnel susceptible de réagir avec le groupe réactif pour former une liaison covalente. Ainsi l'invention concerne aussi l'utilisation des complexes de lanthanides pour le marquage de molécules d'intérêt (protéines, anticorps, enzymes, hormones etc...).

Plus particulièrement, l'invention concerne les conjugués obtenus par réaction entre un complexe selon l'invention comportant un groupe G et une molécule d'intérêt comprenant un groupe fonctionnel, ledit groupe fonctionnel formant une liaison covalente avec l'un des atomes du groupe G, ladite molécule d'intérêt étant choisie parmi : un acide aminé, un peptide, une protéine, un anticorps, un sucre, une chaîne glucidique, un nucléoside, un nucléotide, un oligonucléotide, un substrat d'enzyme (en particulier un substrat d'enzyme suicide telle qu'une benzylguanine ou une benzylcytosine (substrats des enzymes commercialisées sous les dénominations Snaptag et Cliptag)), un chloroalcane (substrat de l'enzyme commercialisée sous la dénomination Halotag), le coenzyme A (substrat de l'enzyme commecialisée sous le nom ACPtag ou MCPtag).

### SYNTHESE

La stratégie générale concernant la préparation des agents complexants (ligands) et des complexes selon l'invention est décrite de manière schématique ci-après (schéma 1 : mono-antenne et schéma 2 : di-antenne), et de manière plus détaillée dans la partie expérimentale.

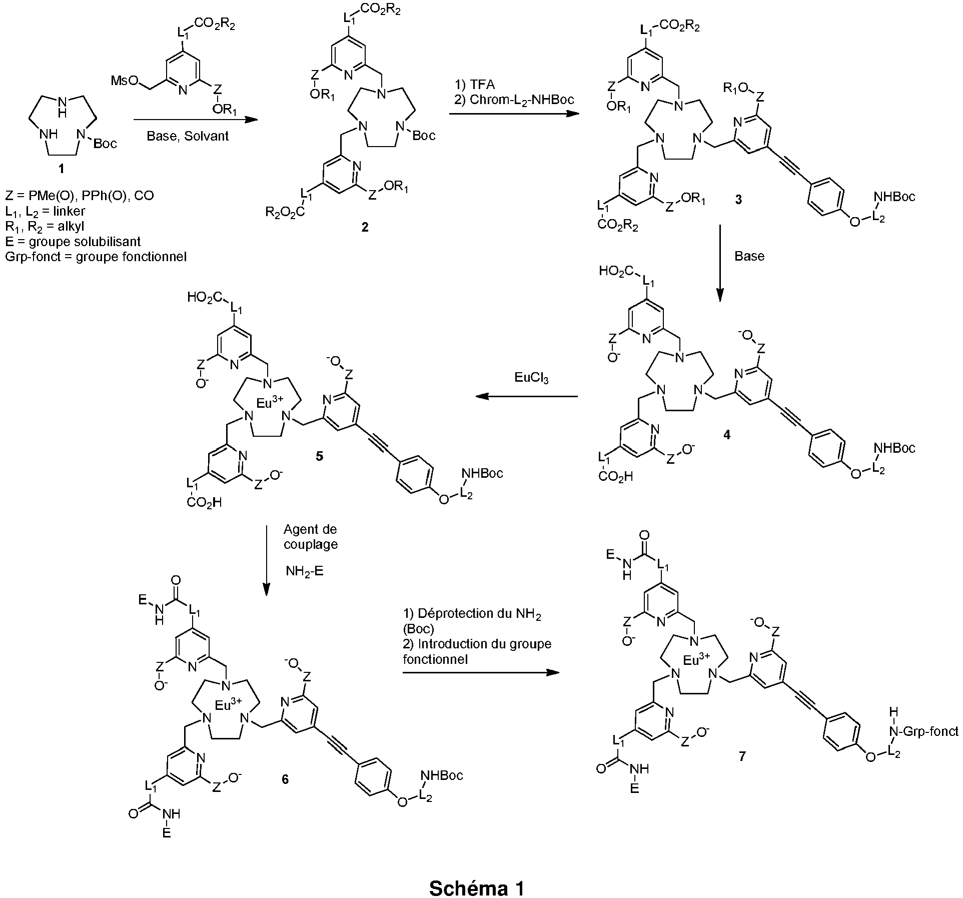

A partir du macrocycle triazacyclononane mono protégé Boc **1,** ont été introduits les deux motifs pyridinyles qui seront utilisés pour accrocher les deux groupements solubilisants. Le groupement protecteur Boc est supprimé puis l'antenne (chromophore) est ajoutée sur le macrocycle conduisant ainsi au ligand **3.** L'hydrolyse des esters (carboxylates et phosphinates) a été réalisée de manière classique en utilisant des conditions basiques. Ceci permet ensuite d'incorporer l'atome d'europium formant ainsi les complexes **5.** A partir de ces complexes, sont introduites les deux fonctions hydrosolubilisantes. Enfin après déprotection du groupement protecteur Boc porté par l'antenne (chromophore), les complexes sont fonctionnalisés (7) afin qu'ils puissent être conjugués sur des biomolécules.

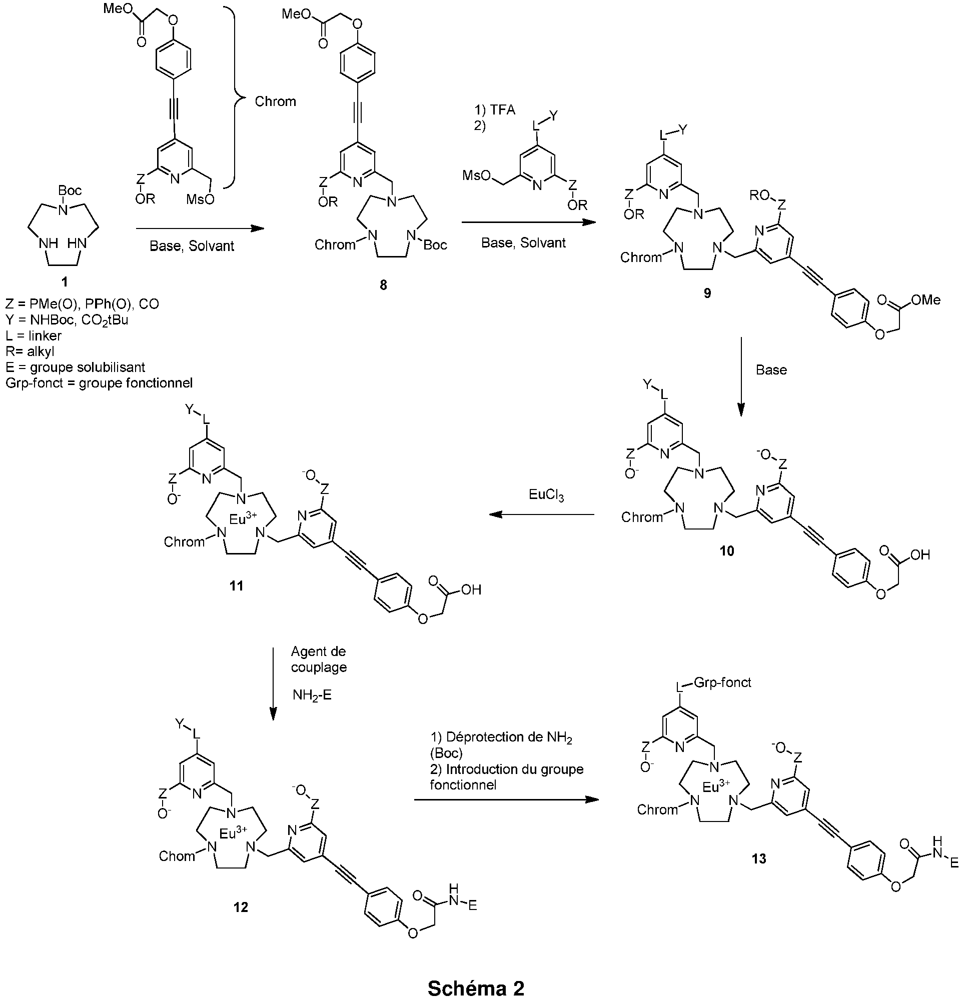

Les systèmes di-antennes sont obtenus en utilisant une stratégie analogue mais en inversant l'ordre d'introduction des motifs pyridinyles et des chromophores. Cette fois les antennes sont introduites en premier lieu pour conduire aux composés **8**. Après suppression du groupement Boc, le dernier motif pyridinyle a été introduit. La suite est identique à savoir hydrolyse des fonctions esters (carboxylates et phosphinates), formation du complexe d'europium, introduction des deux fonctions hydrosolubilisantes (cette fois ces fonctions sont portées par les chromophores) puis incorporation du groupement fonctionnel conduisant ainsi à la famille di-antenne **13**.

### 1) Préparation des briques pyridinyles

Les schémas ci-après (3-11) décrivent les différentes voies synthétiques permettant de disposer de dérivés pydininyles trifonctionnels :
- en position 2 la fonction complexante (acide carboxylique ou acide phosphinique),
- en position 4 une fonction qui permet soit d'introduire le groupe hydrosolubilisant (fonction ester méthylique) ou bien une fonction qui permet d'incorporer le groupe fonctionnel (fonction amine protégée et fonction ester tert-butylique)
- et enfin en position 6 une fonction méthyle alcool qui est convertie en mésylate correspondant afin de pouvoir réagir avec les amines du cycle TACN.

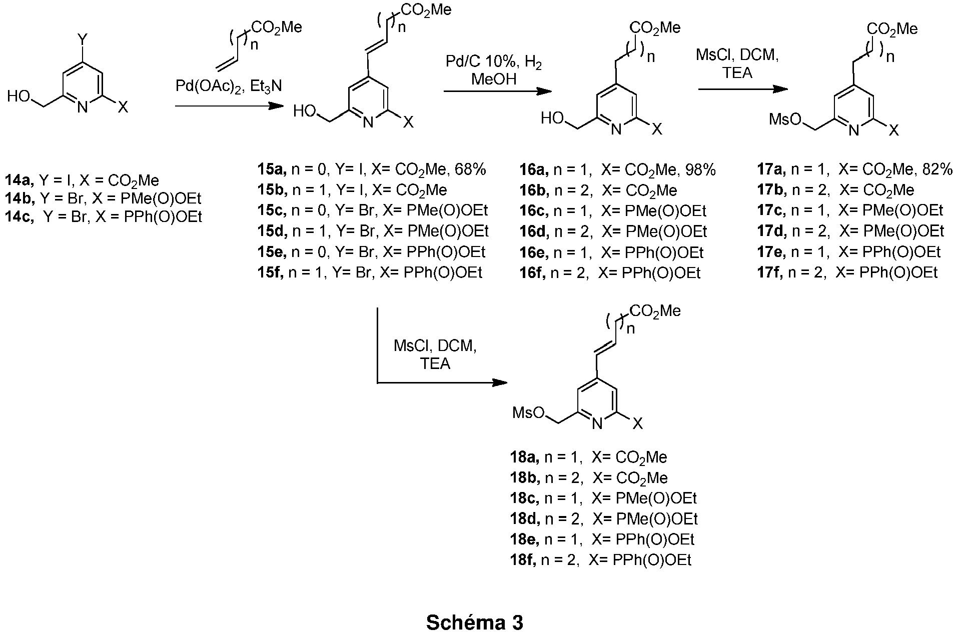

Les synthèses des synthons **14a-c** ont été décrites précédemment (cf. demandes WO 2013/011236 et WO 2014/111661). A partir de ces synthons, la série des composés **17a-f** a été obtenue par une séquence de trois réactions : réaction de Heck permettant de créer la liaison carbone-carbone entre le dérivé de la pyridine et l'alcène. Cette procédure a été décrite par exemple dans la demande de brevet EP-A-2 002 836. La réduction de la double liaison par hydrogénation catalytique suivie de la réaction de mésylation conduit aux composés **17a-f.** Alternativement la double liaison peut être conservée pour rigidifier le système et imposer une orientation apicale aux groupements hydrosolubilisants **(18a-f)**.

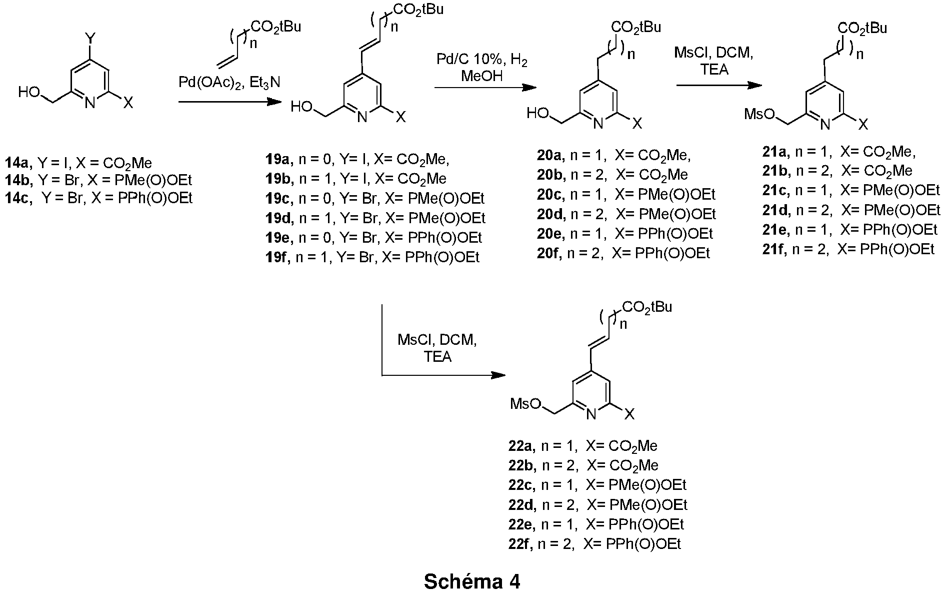

Les composés 21a-f et 22a-f (schéma 4) sous forme ester tert-butylique (analogues de la série 17 et 18) ont été obtenus en suivant la même stratégie et en utilisant l'alcène correspondant.

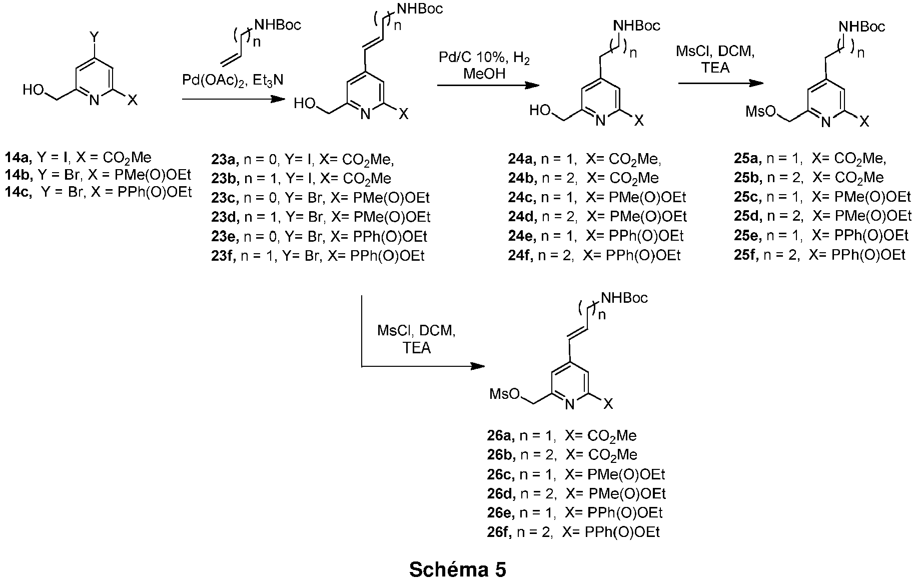

Les composés 25a-f et 26a-f (schéma 5) sous forme NHBoc (analogues de la série 17 et 18) ont été obtenus en suivant la même stratégie en utilisant l'alcène correspondant.

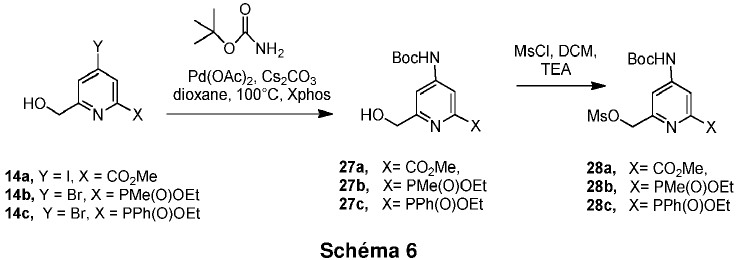

Les composés 28a-c (sans chaîne carbonée) analogues de la série 25 ont été préparés selon une stratégie analogue. L'introduction du groupement NHBoc a été réalisée par exemple en utilisant la méthode décrite dans l'article de revue Tetrahedron Letters 2010, 51, 4445.

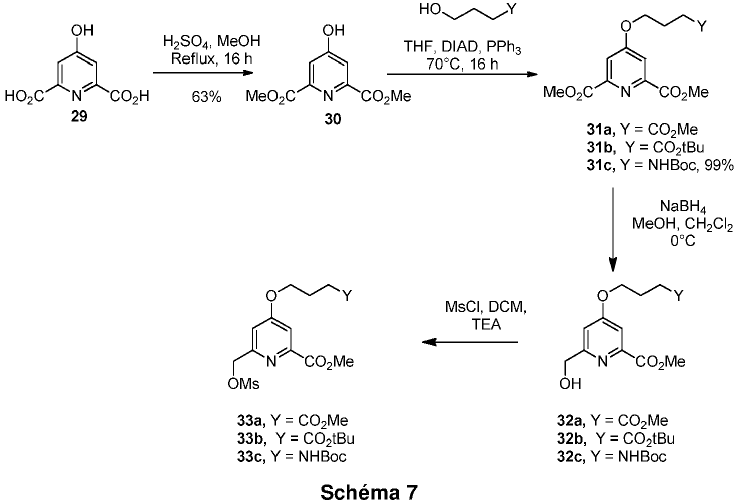

Les dérivés pyridinyles sur lesquels sont intercalés en position 4 un atome d'oxygène entre le linker aliphatique portant la fonction (CO₂R ou NHBoc) et le noyau aromatique (pyridine), ont été préparés selon la méthode décrite dans le schéma 7. L'acide chélidamique **29** a été estérifié sous forme de diester méthylique puis le linker portant la fonction a été introduit en utilisant une réaction de Mitsunobu (procédure décrite par exemple dans Organic Biomolecular Chemistry 2012, 10, 9183). La mono-réduction en utilisant du borohydrure de sodium permet d'obtenir les composés **32a-c** sous forme de monoalcool qui sont ensuite convertis en dérivés mésylés correspondants **33a-c.**

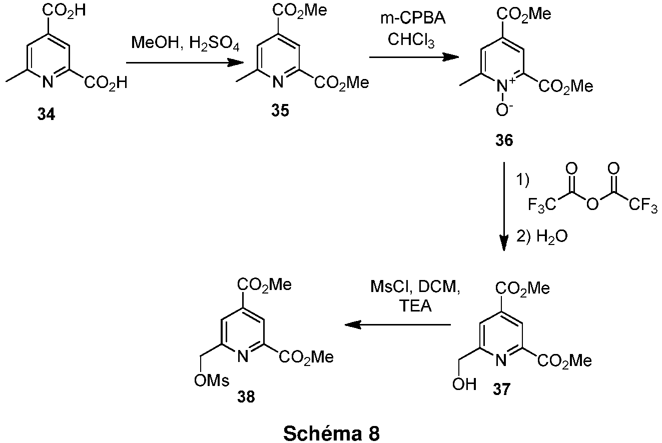

La fonction ester méthylique en position 4 peut être fixée directement sur le noyau aromatique (pyridine). Dans ce cas, il est nécessaire de partir du composé 34 commercial qui est d'abord estérifié. La pyridine est oxydée ensuite en présence de m-CPBA conduisant au dérivé N-oxyde correspondant 36. La fonction N-oxyde réagit facilement avec l'anhydride trifluoroacétique qui subit un réarrangement pour conduire après hydrolyse à la fonction méthyle alcool en position 6. Ce dernier est mésylé dans les conditions classiques conduisant ainsi au composé 38.

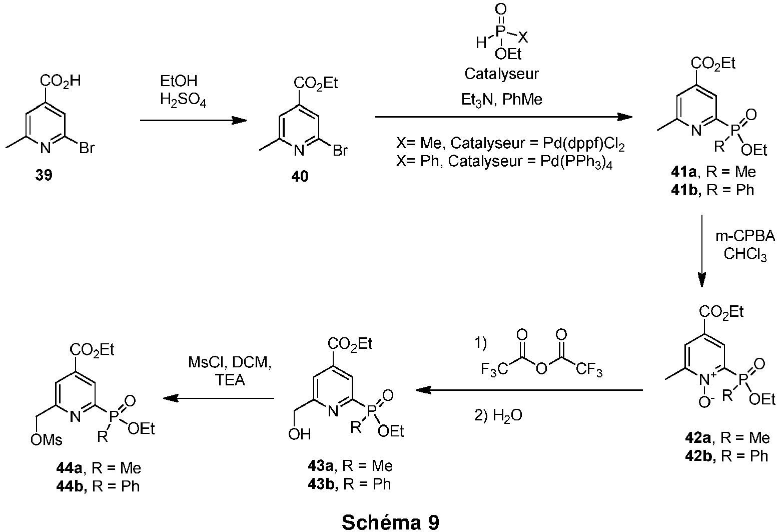

Les dérivés phosphinates 44a-b analogues ont été préparés en utilisant le composé 39 qui est d'abord estérifié puis converti en ester de phosphinate **41a-b.** La suite de la séquence réactionnelle est identique à celle utilisée pour la synthèse du composé **38.**

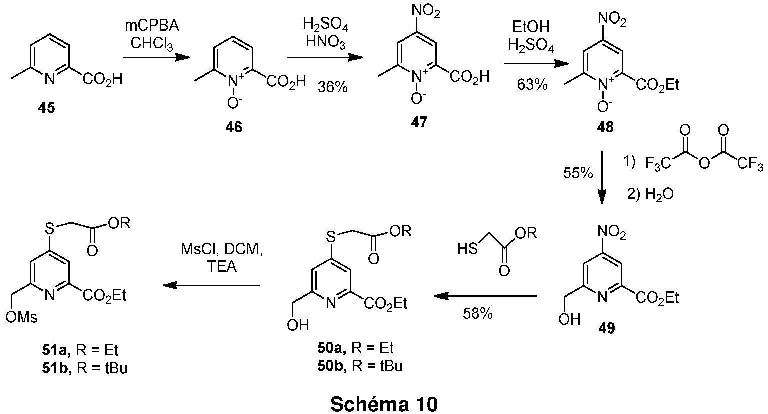

Les dérivés **51a-b** ont été préparés selon la séquence réactionnelle décrite dans le schéma 10. Dans cet exemple les fonctions ester sont introduites en utilisant le thioglycolate d'éthyle ou de tert-butyle.

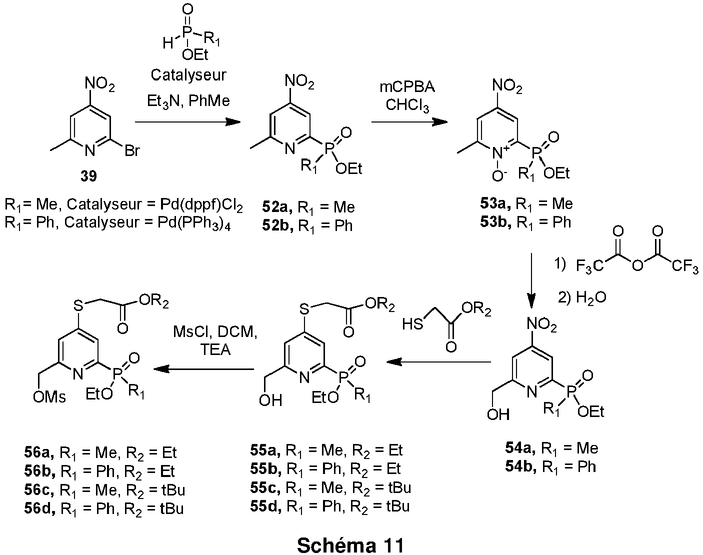

Les analogues phosphinates 56a-d sont préparés selon la voie de synthèse décrite dans le schéma 11.

### 2) Préparation des chromophores

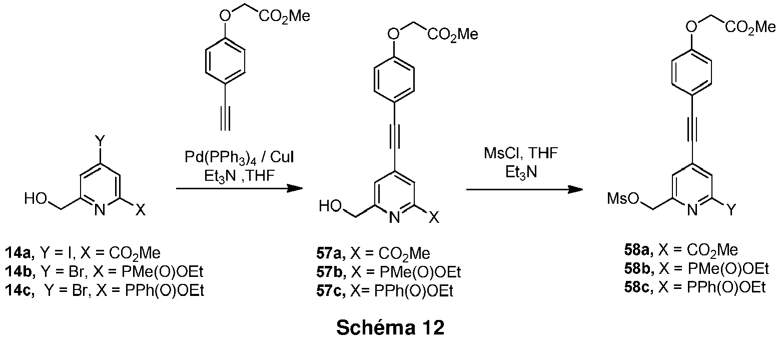

Les chromophores 58a-c (schéma 12) et 60a-c (schéma 13) ont été préparés selon les protocoles détaillés dans les demandes WO 2013/011236 et WO 2014/111661.

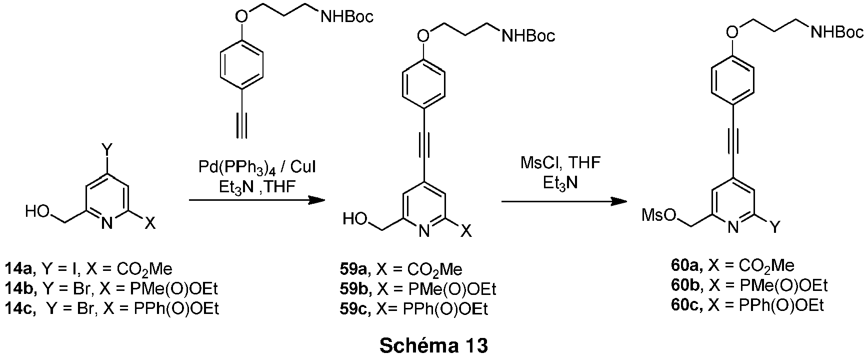

### 3) Synthèse des complexes mono-antenne

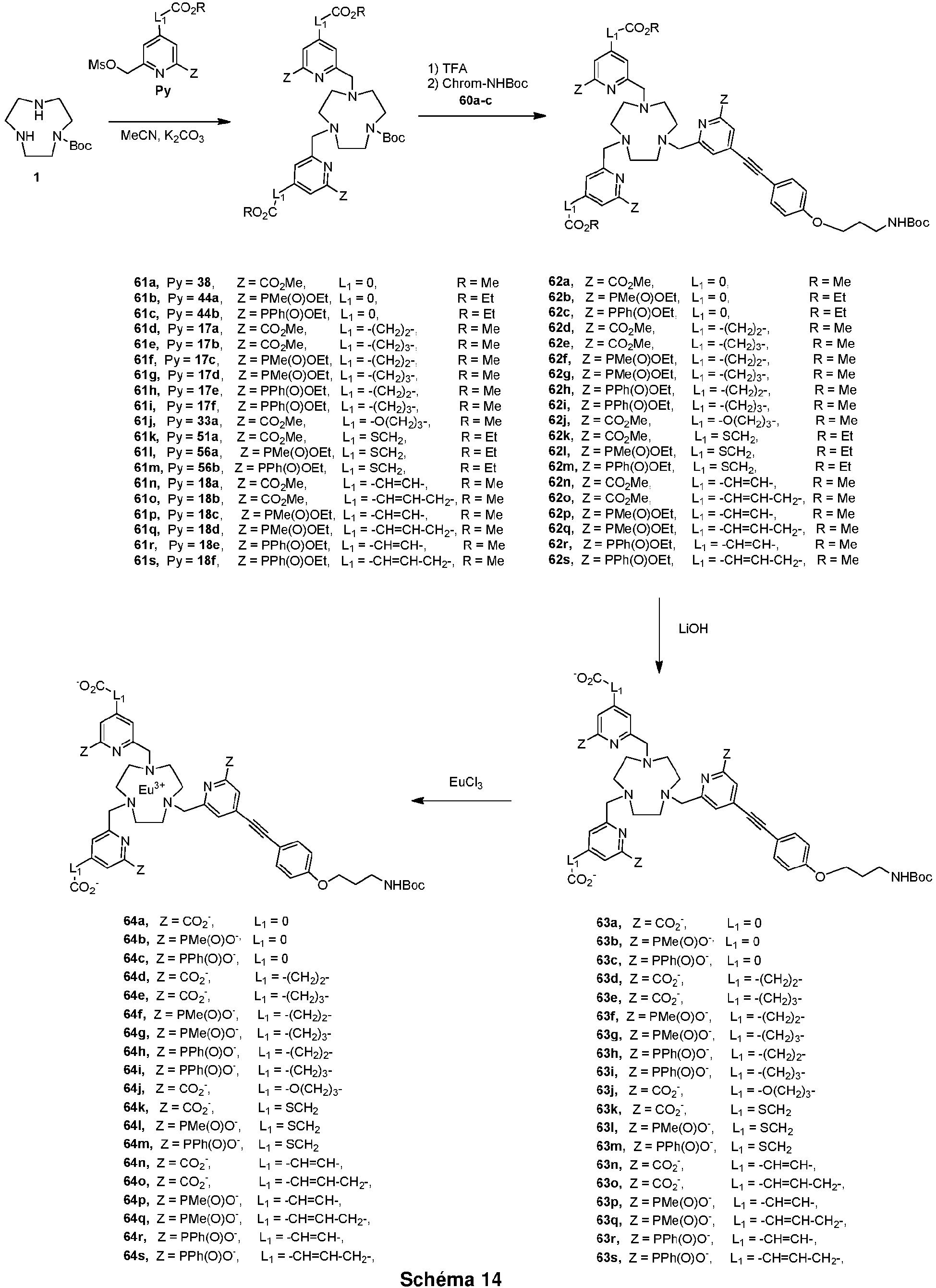

A partir du macrocycle TACN mono protégé Boc sont condensés les dérivés pyridinyles (Py) conduisant aux composés **61a-s**. Le macrocycle est déprotégé et le chromophore correspondant (Z identiques à ceux portés par les Py) est introduit sur le ligand. Les fonctions esters sont hydrolysées (série 63) et l'europium est complexé dans les différents ligands pour conduire à la série des complexes 64a-s.

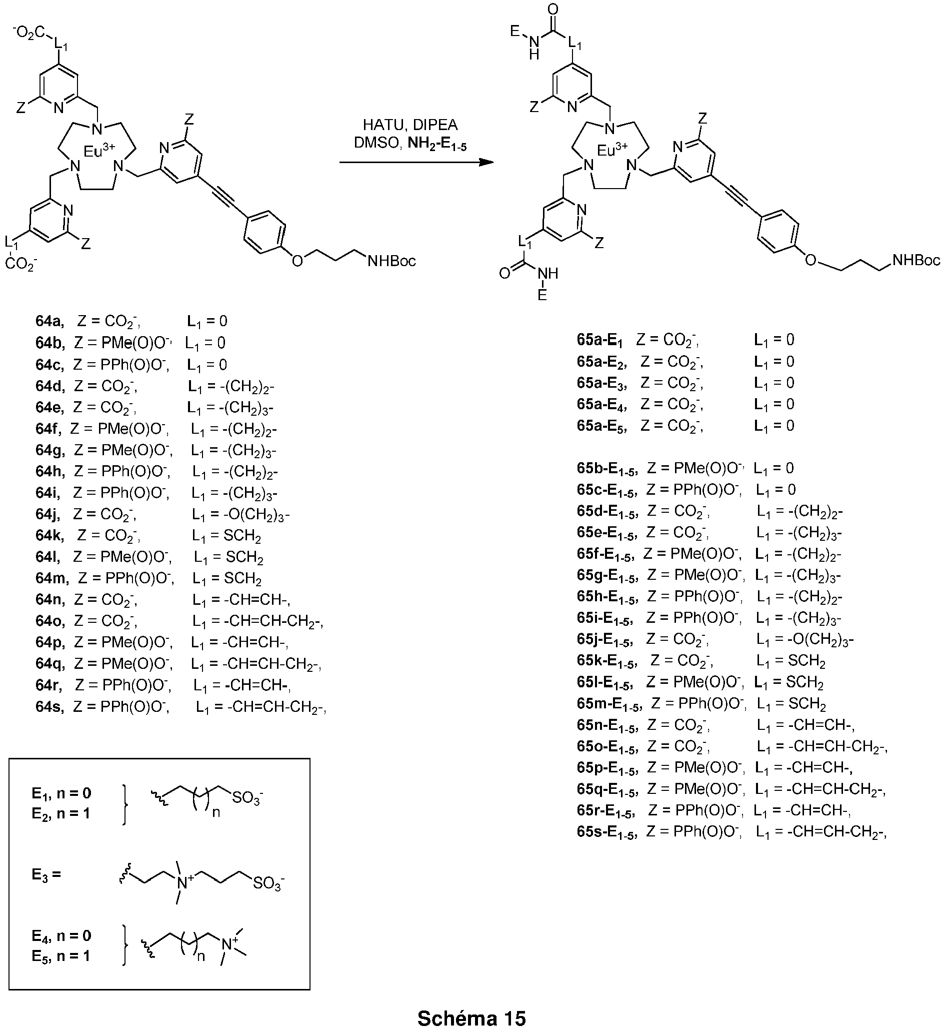

Sur la série 64a-s, les composés sont rendus solubles dans les milieux aqueux par l'introduction de deux groupements hydrosolubilisants : ces groupements sont soit de nature anionique (sulfonates) soit neutre (zwitterion : sulfo-bétaïnes), soit de nature cationique (ammonium quaternaire).

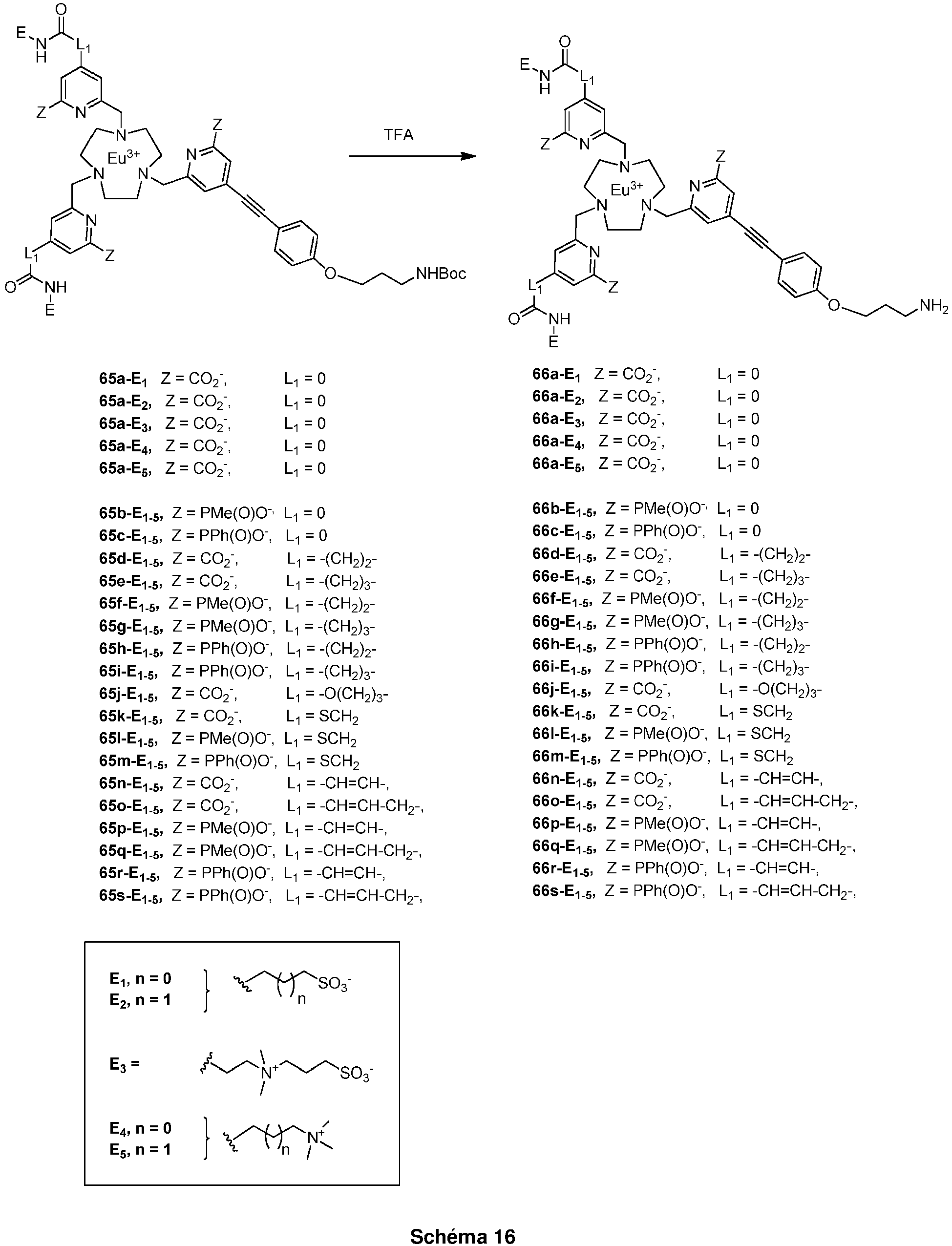

Enfin le groupement Boc est éliminé en présence d'acide trifluoroacétique ce qui conduit aux complexes de l'invention qui sont fonctionnalisés NH₂**(66).**

### 4) Synthèse des complexes di-antennes

La synthèse des complexes di-antennes est décrite dans les shémas 17-20.

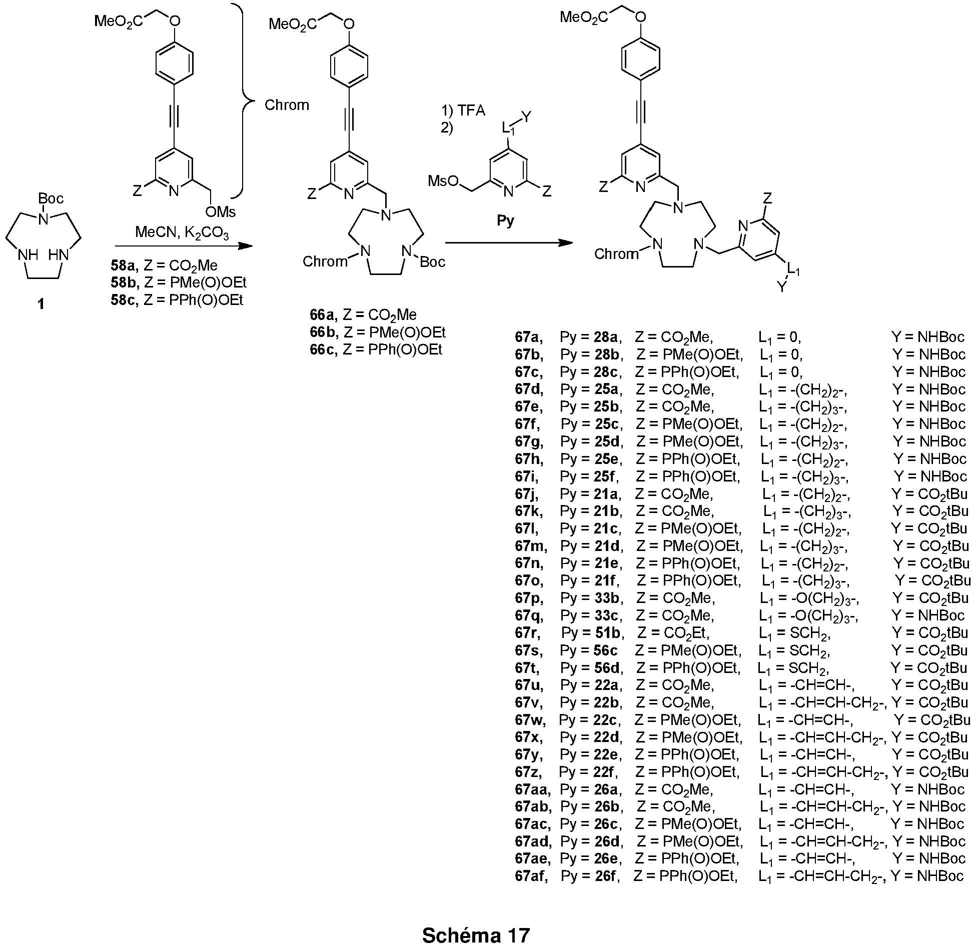

La synthèse commence par la réaction d'alkylation sur le TACN monoprotégé avec les trois types de chromophores : carboxylate, méthyl phosphinate et phényl phosphinate. Le groupement protecteur Boc est éliminé et les pyridines correspondantes portant les Z identiques aux chromophores sont introduites sur le dernier site d'alkylation du TACN conduisant aux composés 67a-af.

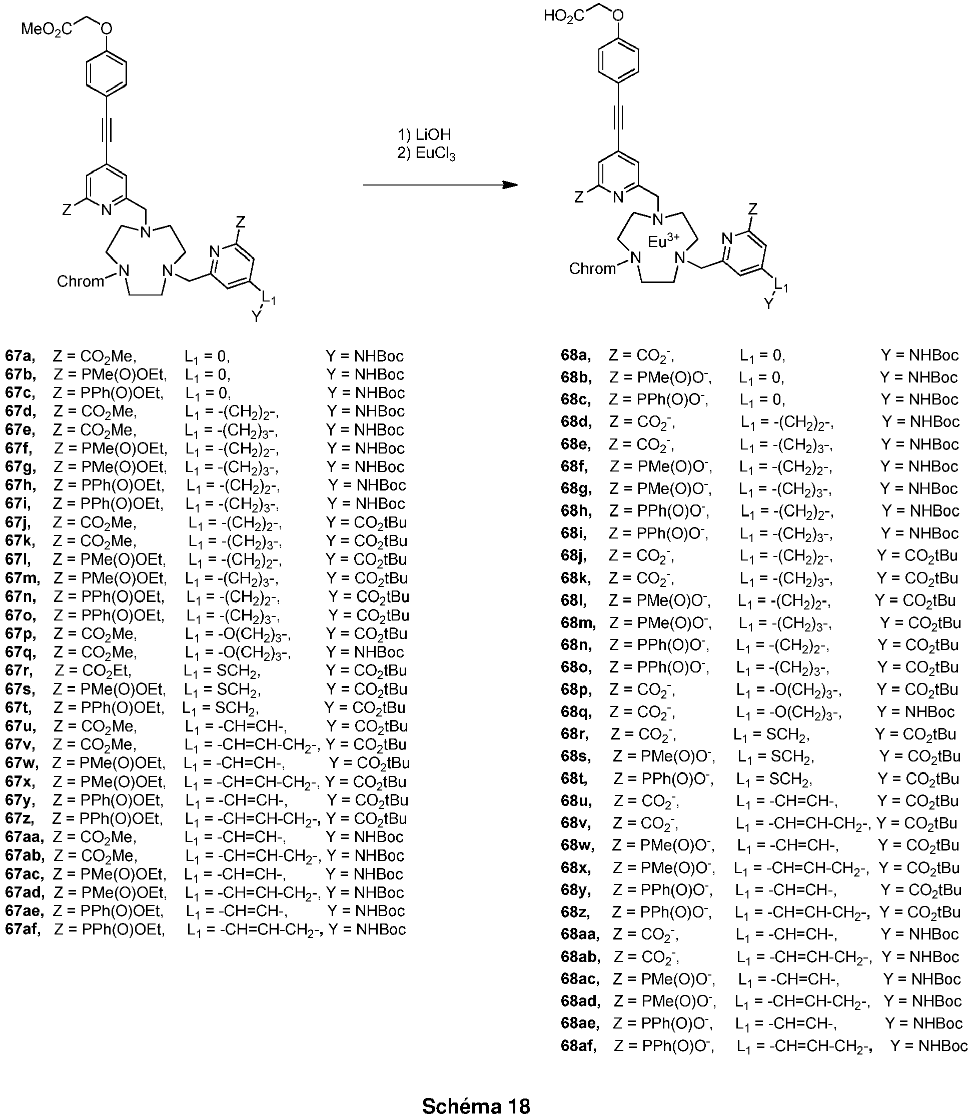

Les ligands sont hydolysés et l'atome d'europium est introduit dans le macrocycle conduisant à la série **68.**

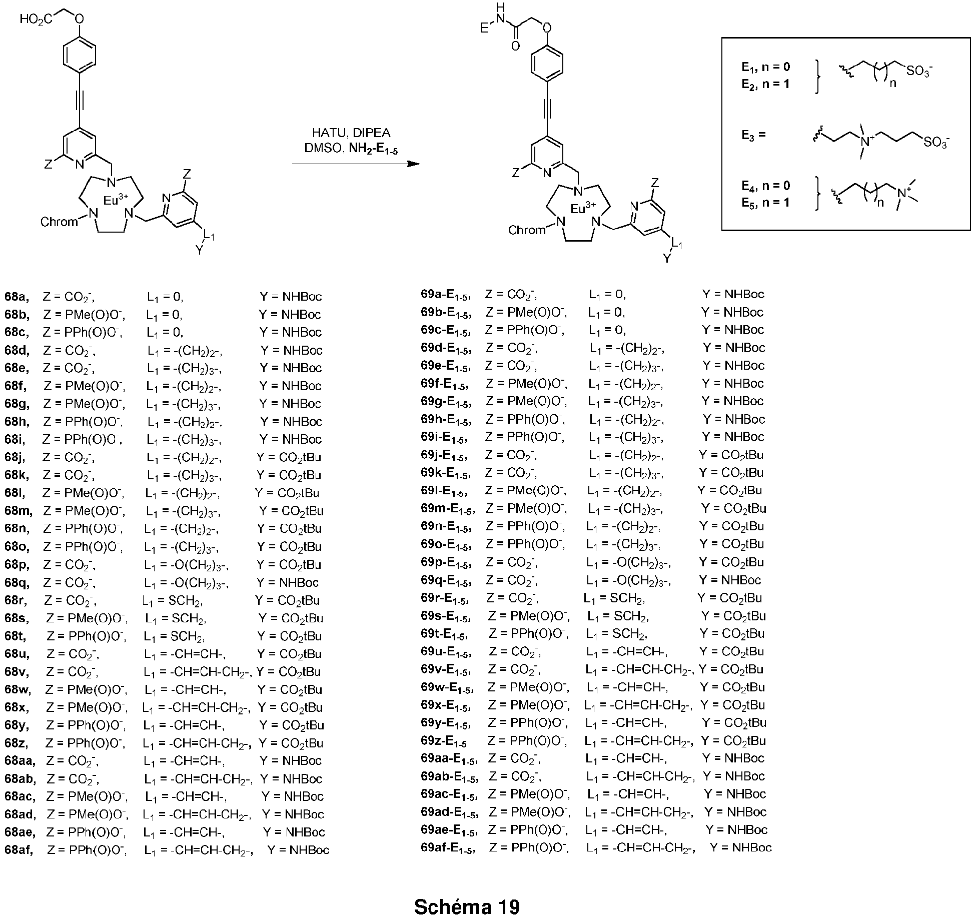

Les groupements hydrosolubilisants (**E₁-E₅**) sont ensuite introduits sur les deux chromophores (schéma 19). Ils sont de natures anioniques, neutres ou cationiques.

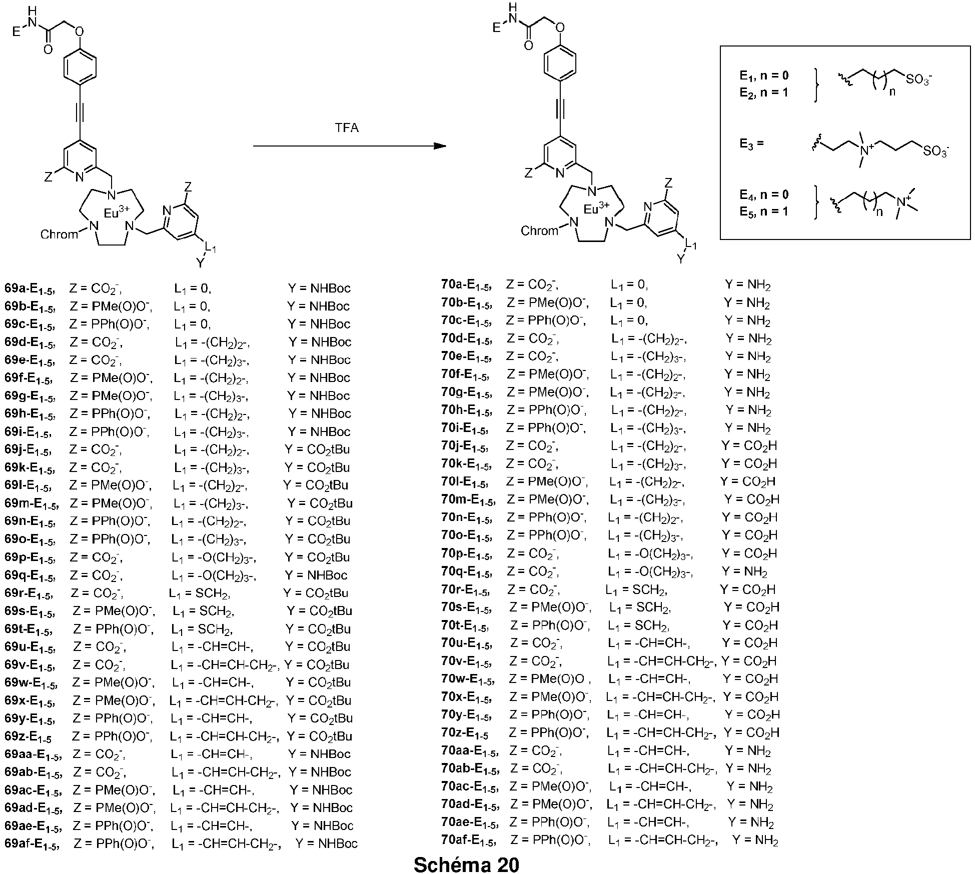

Pour finir le groupement Boc ou ester de *tert*-butyle est ensuite éliminé en présence d'acide trifluoroacétique pour conduire aux composés **70a-af** (schéma 20).

### PARTIE EXPERIMENTALE

### Abréviations utilisées :

AcOEt : acétate d'éthyle
AcOH : acide acétique
Boc : tert-butyloxycarbonyle
CCM : chromatographie en couche mince
CDCl₃ : chloroforme deutéré
CHCl₃ : chloroforme
CsCO₃ : carbonate de césium
CuI : iodure de cuivre(l)
CH₂Cl₂/DCM : dichlorométhane
DIAD : azodicarboxylate de diisopropyle
DIPEA: diisopropyléthylamine
DMF : diméthylformamide
DMSO : diméthylsulfoxyde
Et : éthyle
Et₃N/TEA : triéthylamine
ESI + : ionisation par électronébulisation en mode positif
EtOH : éthanol
J : jour
HATU : (O-(7-azabenzotriazol-1-yl)-N,N,N',N'-tétraméthyluronium hexafluorophosphate)
H₂O : eau
H₂SO₄ : acide sulfurique
HNO₃ : acide nitrique
HPLC : chromatographie liquide à haute performance
LC-MS : chromatographie liquide à haute performance couplée à la spectrométrie de masse
LiOH / Lithine : hydroxyde de lithium
LnCl₃ : chlorure de lanthanide
m-CPBA : acide métachloroperbenzoïque
Me : méthyle
MeCN : acétonitrile
MeOH : méthanol
MgSO₄ : sulfate de magnésium
Ms : mésyle
MsCl : chlorure de mésyle
NaCl : chlorure de sodium
NaH : hydrure de sodium
Pd(dppf)Cl₂ : bis(diphénylphosphino)ferrocène]dichloropalladium(II)
Pd(PPh₃)₄ : tétrakis(triphénylphosphine)palladium(0)
Pd/C : Palladium sur charbon
Pd(OAc)₂ : acétate de palladium (II)
Ph: phényle
PPh₃ : triphénylphosphine
PtF: point de fusion
Py : pyridine
Rf : front de solvant
Rt : temps de rétention
TA : température ambiante
tBu : tertiobutyle
TEA : triéthylamine
TFA : acide trifluoroacétique
THF : tétrahydrofurane
TMS : triméthylsilyle
Ts : tosyle
TSTU: tétrafluoroborate de O-(N-Succinimidyl)-1,1,3,3-tétraméthyluronium
UPLC-MS : chromatographie liquide à ultra haute performance couplée à la spectrométrie de masse
Xphos : 2-dicyclohexylphosphino-2',4',6'-triisopropylbiphényle.

### Chromatographie

Les chromatographies sur couches minces ont été effectuées sur des plaques de gel de silice Merck 60 F₂₅₄ sur feuille d'aluminium ou sur des plaques d'oxyde d'aluminium neutre Merck 60 F₂₅₄ (type E) sur feuille d'aluminium.

Les chromatographies liquides à haute performance (HPLC) analytiques et préparatives ont été effectuées sur deux appareils :
- HPLC Analytique: ThermoScientific, pompe quaternaire P4000, Détecteur UV 1000 à lampe au deutérium (190-350 nm), colonne analytique Waters XBridge C₁₈, 3,5 µm, 4,6 x 100 mm.
- HPLC Préparative : Shimadzu, 2 pompes LC-8A, détecteur UV à barrette de diodes Varian ProStar, colonne préparative Waters XBridge prép. C18, 5 µm: 19 x 100 mm ou 50 x 150 mm.

Les chromatographies liquides à Ultra-haute performance (UPLC) analytiques ont été réalisées sur un appareil Waters Acquity HClass avec comme détecteur soit un detecteur UV à barrette de diode de type PDA, soit un détecteur de masse simple quadripolaire de type SQD2. La sonde utilisée est un électro-spray en mode positif : tension de capillaire à 3,2 KV - tension de cône à 30 V.

Les chromatographies sur colonne de silice ont été réalisées sur gel de silice Merck 60 (0.040-0.063 mm). Les chromatographies sur colonne d'alumine ont été réalisées sur oxyde d'aluminium Sigma-Aldrich, neutre, activé, Brochmann I.

### Gradient A

Colonne Waters Acquity C₁₈, 300 Å, 1,7 µm, 2,1 x 50 mm - A / eau 0,1% acide formique B / acétonitrile 0,1% acide formique t = 0 min 5 % B - t = 0,2 min 5 % B - t = 5 min 100 % B - 0,6 mL.min⁻¹.

### Gradient B

Colonne Waters Xbridge C₁₈, 5 µm, 50 x 150 mm - A / eau 25 mM TEAAc pH 7 B / acétonitrile t = 0 min 10 % B ― t = 19 min 60 % B - 100 mL.min⁻¹.

### Gradient C

Colonne Waters Acquity C₁₈, 300 Å, 1,7 µm, 2,1 x 50 mm - A / eau 5 mM acétate d'ammonium B / acétonitrile t = 0 min 5 % B ― t = 0,2 min 5 % B ― t = 5 min 100 % B ― 0,6 mL.min⁻¹.

### Gradient D

Colonne Waters Xbridge C₁₈, 5 µm, 20 x 100 mm - A / eau 25 mM TEAAc pH 7 B / acétonitrile t = 0 min 5 % B ― t = 19 min 60 % B ― 20 mL.min⁻¹.

### Gradient E

Colonne Waters Xbridge C₁₈, 5 µm, 20 x 100 mm - A / eau 25 mM TEAAc pH 7 B / acétonitrile t = 0 min 2 % B ― t = 19 min 40 % B ― 20 mL.min⁻¹.

### Gradient F

Colonne Waters Xbridge C₁₈, 5 µm, 20 x 100 mm - A / eau 25 mM TEAAc pH 6 B / acétonitrile t = 0 min 2 % B ― t = 19 min 40 % B ― 20 mL.min⁻¹.

### Spectroscopie

- Résonance magnétique nucléaire
   Les spectres RMN (¹H, ¹³C et ³¹P) ont été réalisés à l'aide d'un spectromètre Bruker Avance 400 MHz NanoBay (aimant de 9,4 Teslas), muni d'une sonde de mesure BBFO, multi noyaux de diamètre de 5 mm, de gradient Z et de lock²H. Les déplacements chimiques (δ) sont exprimés en partie par million (ppm). Les abréviations suivantes sont utilisées :
   s : singulet, se : singulet élargi, d : doublet, t : triplet, q : quadruplet, m : multiplet, dd : doublet dédoublé, td : triplet dédoublé, qd : quadruplet dédoublé, ddd : doublet de doublet dédoublé.
- Spectrométrie de masse (LRMS)
   Les spectres de masse (LC-MS) ont été réalisés à l'aide d'un spectromètre Waters ZQ 2000 simple quadipôle à source multimode ESI/APCI équipé de colonne Waters XBridge C₁₈, 3,5 µm, 4,6 x 100 mm ou bien un spectromètre de masse simple quadripolaire de type SQD2.
- Spectrométrie de masse haute résolution (HRMS)
   Les analyses ont été effectuées avec un spectromètre de masse QStar Elite (Applied Biosystems SCIEX) équipé d'une source d'ionisation à pression atmosphérique (API) assistée pneumatiquement. L'échantillon a été ionisé en mode electrospray positif dans les conditions suivantes : tension electrospray (ISV) : 5500 V ; tension d'orifice (OR) : 20 V ; pression du gaz de nébulisation (air) : 20 psi. Le spectre de masse haute résolution (HRMS) a été obtenu avec un analyseur temps de vol (TOF). La mesure de masse exacte a été effectuée en triplicata avec un double étalonnage interne.

**Exemples** :
**Composé 1** : le composé 1 a été préparé selon la procédure décrite dans les demandes WO 2013/011236 et WO 2014/111661.
**Composés 14a-14c** : les composés **14a-14c** ont été préparés selon la procédure décrite dans les demandes WO 2013/011236 et WO 2014/111661.
**Composé 15a** : dans un ballon de Schlenk de 100 mL le composé **14a** (440 mg, 1,5 mmol) a été solubilisé dans du DMF anhydre (10 mL) pour donner une solution incolore. Au mélange réactionnel a été ajouté la tri(o-tolyl)phosphine (91 mg, 0,3 mmol), Pd(OAc)₂ (33,7 mg, 0,15 mmol), la TEA (0,314 ml, 2,252 mmol) puis l'acrylate de méthyle (0,203 mL, 2,252 mmol) en une seule fois. La réaction a été agitée à 70 °C pendant 5 h. L'avancement de la réaction a été suivi par UPLC-MS (gradient A). Après cette période, la réaction était totale. Le mélange réactionnel a été concentré sous pression réduite, dilué dans AcOEt (50 mL), lavé avec de l'eau (2 x 50 mL) puis de l'eau saturée en NaCl (50 mL). La phase organique a été séchée sur MgSO₄, filtrée et concentrée sous pression réduite. Le brut a été purifié par chromatographie sur colonne de silice en utilisant un gradient de solvant DCM/MeOH de 100/0 jusqu'à 99/1 pour conduire au composé **15a** (233 mg, 62%) sous forme de poudre blanche. Pf = 156,4-156,9°C - HPLC gradient A - Rt = 2,03 min - [M+H]⁺, m/z 251,9 - Rf = 0,41 (silice, dichlorométhane - méthanol 96 : 4 - HRMS (ESI+) calculée pour C₁₂H₁₄NO₅⁺ [M+H]⁺, m/z 252,0866 , trouvée : 252,0868 - RMN ¹H (400 MHz, CDCl₃) δ : 8,13 (s, 1 H, Py H⁵), 7.67 (d, J = 16.2 Hz, 1H), 7,65 (s, 1 H, Py H³), 7,19 (dd, J = ; 16,2 Hz, 2 H, HC=CH), 6.71 (d, J = 16.2 Hz, 1H), 4,91 (s, 2 H, CH₂-OH), 4,04 (s, 3 H, Py-COOMe), 3,85 (s, 3 H, COOMe), 3,49 (s I, 1 H, OH) ; RMN ¹³C (100 MHz, CDCl₃) δ : 166,20 (COOMe), 165,24 (Py-COOMe), 161,59 (Py C²), 147,97 (Py-C=C), 143,74 (Py C⁶), 140,92 (Py C⁴), 123,77 (Py C³), 122,08 (Py C⁵), 121,86 (Py-C=C), 64,68 (CH₂-OH), 53,10 (Py-CO₂CH₃), 52,19 (CO₂CH₃).
**Composés 15b-15f** : ces composés ont été préparés selon la même procédure que celle utilisée pour la synthèse de 15a en utilisant les alcènes correspondants.
**Composé 16a** : dans un ballon de 50 mL le composé **15a** (233 mg, 0,927 mmol) a été solubilisé dans du MeOH (10 mL) pour donner une solution incolore. Au mélange réactionnel a été ajouté le Pd/C 10% (23,69 mg, 0,022 mmol) en une seule fois. La réaction a été agitée à TA avec un barbotage de dihydrogène pendant 2 h. L'avancement de la réaction a été suivi par UPLC-MS (gradient A). Après cette période, la réaction était totale. Le mélange réactionnel a été filtré sur filtre nylon 22 µm, évaporé à sec pour conduire au composé **16a** (231 mg, 98%) sous forme de poudre blanche. Pf = 133,2-136,4°C - HPLC gradient A - Rt = 1,86 min - [M+H]⁺, m/z 253,2 - HRMS (ESI+) calculée pour C₁₂H₁₆NO₅⁺ [M+H]⁺, m/z 254,1023 , trouvée : 254,1024 - RMN ¹H (400 MHz, CDCl₃) δ : 7,9 (s, 1 H, Py H⁵), 7,41 (s, 1 H, Py H³), 4,85 (s, 2 H, CH₂-OH), 4,01 (s, 3 H, Py-COOMe), 3,69 (s, 3 H, COOMe), 3,05 (t, J = 7,6 Hz, 2 H, Py-CH₂-CH₂), 2,71 (t, J = 7,6 Hz, 2 H, Py-CH₂-CH₂) ; RMN ¹³C (100 MHz, CDCl₃) δ : 172,41 (COOMe), 165,65 (Py-COOMe), 160,49 (Py C²), 151,67 (Py C⁴), 147,22 (Py C⁶), 140,92 (Py C³), 123,96 (Py C³), 123,9 (Py C⁵), 64,62 (CH₂-OH), 52,91 (Py-CO₂CH₃), 51,91 (CO₂CH₃), 33,97 (Py-CH₂-CH₂), 30,07 (Py-CH₂-CH₂).
**Composés 16b-16f** : ces composés ont été préparés selon la même procédure que celle utilisée pour la synthèse de 16a.
**Composé 17a** : dans un ballon de 100 mL le composé 16a (231 mg, 0,912 mmol) a été solubilisé dans du THF anhydre (30 mL) pour donner une solution incolore. Au mélange réactionnel placé dans un bain de glace a été ajouté la TEA (0,127 mL, 0,912 mmol) puis le MsCl (72 µL, 0,912 mmol) en une seule fois. Le mélange a été réchauffé à TA et agité pendant 15 min. L'avancement de la réaction a été suivi par UPLC-MS (gradient A). Après cette période, la réaction était totale. Le mélange réactionnel a été concentré sous pression réduite, dilué dans du DCM (50 mL), lavé avec de l'eau (2 x 25 mL) puis de l'eau saturée en NaCl (20 mL). La phase organique a été séchée sur MgSO₄, filtrée et concentrée sous pression réduite pour conduire au composé **17a** (249 mg, 82%) sous forme de poudre blanche. HPLC gradient A - Rt = 3,2 min - [M+H]⁺, m/z 332,3 - Rf = 0,23 (silice, dichlorométhane - méthanol 98 : 2 - HRMS (ESI+) calculée pour C₁₃H₁₈NO₇S⁺ [M+H]⁺, m/z 332,0799, trouvée : 332,0799 - RMN ¹H (400 MHz, CDCl₃) δ : 7,98 (s, 1 H, Py H⁵), 7,54 (s, 1 H, Py H³), 5,41 (s, 2 H, CH₂-OMs), 4,00 (s, 3 H, Py-COOMe), 3,69 (s, 3 H, COOMe), 3,16 (s, 3 H, OMs), 3,07 (t, J = 7,5 Hz, 2 H, Py-CH₂-CH₂), 2,72 (t, J = 7,5 Hz, 2 H, Py-CH₂-CH₂) ; RMN ¹³C (100 MHz, CDCl₃) δ : 172,23 (COOMe), 165,27 (Py-COOMe), 154,56 (Py C²), 152,46(Py C⁴), 147,93 (Py C⁶), 125,19 (Py C³), 125,03 (Py C⁵), 70,97 (CH₂-OMs), 53,08 (Py-CO₂CH₃), 51,94 (CO₂CH₃), 38,05 (CH₂-OSO₂CH₃), 33,87 (Py-CH₂-CH₂), 30,07 (Py-CH₂-CH₂).
**Composés 17b-17f** : ces composés ont été préparés selon la même procédure que celle utilisée pour la synthèse de **16a.**
**Composés 18a-18f** : ces composés ont été préparés selon la même procédure que celle utilisée pour la synthèse de **17a.**
**Composés 19a-19f** : ces composés ont été préparés selon la même procédure que celle utilisée pour la synthèse de **15a**.
**Composés 20a-20f** : ces composés ont été préparés selon la même procédure que celle utilisée pour la synthèse de **16a**.
**Composés 21a-21f** : ces composés ont été préparés selon la même procédure que celle utilisée pour la synthèse de **17a.**
**Composés 22a-22f** : ces composés ont été préparés selon la même procédure que celle utilisée pour la synthèse de **17a.**
**Composés 23a-23f** : ces composés ont été préparés selon la même procédure que celle utilisée pour la synthèse de **15a** en utilisant les alcènes correspondants.
**Composés 24a-24f** : ces composés ont été préparés selon la même procédure que celle utilisée pour la synthèse de **16a.**
**Composés 25a-25f** : Ces composés ont été préparés selon la même procédure que celle utilisée pour la synthèse de **17a.**
**Composés 26a-26f** : ces composés ont été préparés selon la même procédure que celle utilisée pour la synthèse de **17a.**
**Composé 27a-c** : les composés **27a-c** ont été préparés selon la procédure décrite dans l'article : Tetrahedron Letters 2010, 51, 4445.
**Composés 28a-28c** : ces composés ont été préparés selon la même procédure que celle utilisée pour la synthèse de **17a.**
**Composé 29** : ce composé est disponible commercialement.
**Composé 30** : le composé **30** a été préparé selon la procédure décrite dans l'article : Dalton Transactions 2010, 39, 707.
**Composés 31a-31c** : les composés 31a-31c ont été préparés selon la procédure décrite dans l'article : Organic Biomolecular Chemistry 2012, 10, 9183.
**Composés 32a-32c** : les composés **32a-32c** ont été préparés selon la procédure décrite dans l'article : Journal of Organic Chemistry 2010, 75, 7175.
**Composés 33a-33c** : les composés **33a-33c** ont été préparés selon la procédure décrite dans l'article : Journal of Organic Chemistry 2010, 75, 7175.
**Composé 34** : ce composé est disponible commercialement.
**Composé 35** : le composé **35** a été préparé selon la procédure décrite dans l'article : Bioorganic Chemistry 2014, 57, 148.
**Composé 36** : le composé **36** a été préparé selon la procédure décrite dans l'article : Carbohydrate Research 2013, 372, 35.
**Composé 37** : le composé **37** a été préparé selon la procédure décrite dans WO 2014/111661. **Composé 38** : le composé a été préparé selon la même procédure que celle utilisée pour la synthèse de **17a.**
**Composé 39** : ce composé est disponible commercialement.
**Composé 40** : le composé **40** a été préparé selon la procédure décrite dans l'article : Bioorganic Chemistry 2014, 57, 148.
**Composé 41a-b** : les composés **41a-b** ont été préparés selon la procédure décrite dans la demande WO 2014/111661 en utilisant le catalyseur correspondant.
**Composé 42a-b** : les composés **42a-b** ont été préparés selon la même procédure que celle utilisée pour la synthèse de **36.**
**Composé 43a-b** : les composés **43a-b** ont été préparés selon la même procédure que celle utilisée pour la synthèse de **37.**
**Composé 44a-b** : les composés **44a-b** ont été préparés selon la même procédure que celle utilisée pour la synthèse de **17a.**
**Composé 45** : disponible commercialement.
**Composé 46** : le composé 46 a été préparé selon la procédure décrite dans l'article : Chemistry - A European Journal, 2014, 20, 3610.
**Composé 47 :** le composé **46** (0,313 g, 2,04 mmol) a été dissous dans H₂SO₄ (11 mL) à TA puis la solution a été refroidie dans un bain de glace. A ce mélange a été ajouté goutte à goutte HNO₃ (9,7 mL) et la solution a été chauffée à 100 °C pendant 2 j. Le mélange a été refroidi à TA puis versé dans de la glace pilée (100 g). Après 1 h, la phase aqueuse a été extraite avec du CH₂Cl₂ (3 x 50 mL), les phases organiques ont été regroupées, séchées sur MgSO₄ et le produit brut a été purifié par chromatographie sur colonne de silice en utilisant un mélange de solvant (CH₂Cl₂-AcOH, 98/2) pour conduire à un solide blanc (224 mg, 56%). *R_{f}*(CH₂Cl₂/AcOH, 98/2) = 0.38; PtF: 147°C; RMN ¹H (400 MHz, CDCl₃, δ): 16.49 (s, 1H, COOH), 9.08 (s, 1H, H³), 8.36 (s, 1H, H⁵), 2.75 (s, 3H, py-CH₃); RMN ¹³C (101 MHz, CDCl₃, δ): 159.4 (COOH), 152.4 (C⁶), 144.4 (C⁴), 138.7 (C²), 123.1 (C⁵), 121.7 (C³), 18.4 (py-CH₃); MS Calculée pour C₇H₇N₂O₅ 199,036. Trouvée 199,035 [M+H]⁺.
**Composé 48:** le composé **47** (2,9, 14,7 mmol) a été dissous dans du MeOH anhydre (3 mL) à TA. A cette solution a été ajouté H₂SO₄ (200 µL) goutte à goutte et la solution a été chauffée à 65°C pendant 3 j. La solution a été refroidie à TA et le solvant a été éliminée sous pression réduite. Au residu a été ajoutée H₂O (30 mL) et la solution a été extraite avec AcOEt (3 x 20 mL). Les phases organiques ont été réunies, lavée avec une solution de bicarbonate de sodium à 5% (2 x 20 mL), puis avec une solution de saumure saturée (20 mL). Après séchage sur MgSO₄, le solvant a été filtré, éliminé sous pression réduite pour conduire au composé **48** qui a été utilisée dans la suite de la synthèse sans purification supplémentaire (57 mg, 76%). RMN ¹H (400 MHz, CDCl₃, δ): 8.33 (d, 1H, ⁴J 3.1, H⁵), 8.19 (d, 1H, ⁴J 3.1, H³), 4.02 (s, 3H, CH₃CO), 2.57 (s, 3H, py-CH₃); RMN ¹³C (100 MHz, CDCl₃, δ): 160.8 (COOMe), 152.7 (C⁶), 142.1 (C⁴), 140.5 (C²), 121.4 (C⁵), 119.3 (C³), 53.8 (OCH₃), 18.3 (py-CH₃); MS Calculée C₈H₉N₂O₅ 213,051. Trouvée 213,050 [M+H]⁺.
**Composé 49 :** à une solution de composé **48** (114 mg, 0,54 mmol) dans du CHCl₃ (10 mL) a été ajouté à TA de l'anhydride trifluoroacétique (1,48 mL, 10,8 mmol). Le mélange a été chauffé à 60°C pendant 5 h sous atmosphère inerte. Après cette période, la réaction a été refroidie à TA puis le solvant a été éliminé sous pression réduite. A l'huile jaune, ont été ajoutés EtOH (3 mL) et H₂O (3 mL) et la solution a été agitée à TA pendant 2 h. Les solvants ont été éliminés sous pression réduite et la phase aqueuse a été extraite avec du CH₂Cl₂ (3 x 30 mL). Les phases organiques ont été réunies, séchées sur MgSO₄ et évaporées sous pression réduite. Le résidu a été purifié par chromatographie sur colonne de silice en utilisant un gradient de solvant Hexane/AcOEt, 70/30 à 50/50 pour conduire au composé **49** (74 mg, 65%). *R_{f}* (CH₂Cl₂/MeOH, 95/5) = 0.67; RMN ¹H (400 MHz, CDCl₃, δ): 8.68 (d, 1H, ⁴J 2.1, H³), 8.37 (d, 1H, ⁴J 2.1, H⁵), 5.06 (s, 2H, C*H₂*OH), 4.06 (s, 3H, CH₃CO); RMN ¹³C (100 MHz, CDCl₃, δ): 164.3 (COOMe), 163.6 (C⁶), 155.3 (C⁴), 149.7 (C²), 116.4 (C⁵), 116.3 (C³), 64.5 (CH₂OH), 29.5 (CO₂CH₃).
**Composé 50a:** à une solution de composé **49** (21,6 mg, 0,102 mmol) dans du DMF anhydre (1 mL) ont été ajoutés du NaH (17 mg, 0.708 mmol) et du thioglycolate d'éthyle (35 µL, 0.320 mmol) sous atmosphère inerte et à TA. Le mélange a été agité à TA pendant 2 h sous atmosphère inerte. Le solvant a été ensuite éliminé sous pression réduite et à l'huile jaune ont été ajoutés du MeOH (5 mL) et H₂SO₄ (200 µL). La solution a été chauffée à 65°C pendant 72 h sous argon. Le solvant a été éliminé sous pression réduite et H₂O (10 mL) a été ajouté au résidu, et la solution aqueuse a été extraite avec AcOEt (3 x 20 mL). Les phases organiques ont été rassemblées et séchées sur MgSO₄, filtrées et concentrées sous pression réduite. Le résidu a été purifié par chromatographie sur colonne de silice en utilisant comme éluant CH₂Cl₂-MeOH, 98/2 conduisant ainsi au composé **50a** (8.2 mg, 25%). *R_{f}* (DCM/MeOH, 95/5) = 0.35; RMN ¹H (400 MHz, CDCl₃, δ): 7.88 (d, 1H, ⁴J 1.9, H⁵), 7.63 (d, 1H, ⁴J 1.9, H³), 4.69 (s, 2H, CH₂OH), 4.02 (s, 2H, CH₂S), 3.96 (s, 3H, CH₃CO), 3.76 (s, 3H, CH₃CO); RMN ¹³C (100 MHz, CDCl₃, δ): 170.7 (COOMe), 166.4 (COOMe), 163.3 (C²), 152.3 (C⁴), 147.8 (C⁶), 121.2 (C⁵), 121.1 (C³), 65.1 (CH₂OH), 53.3 (CO₂-CH₃), 48.5 (CO₂-CH₃), 33.6 (SCH₂).
**Composé 50b:** le composé **50b** a été préparé selon la même procédure que celle utilisée pour la synthèse de **50a.**
**Composé 51a:** à une solution de composé **50a** (8,2 mg, 0,03 mmol) dans du THF anhydre (2 mL) ont été ajoutés de la triéthylamine (12,5 µL, 0,09 mmol) et du MsCl (3,5 µL, 0,045 mmol). Cette solution a été agitée à TA pendant 3,5 h. Après cette période, le solvant a été éliminé sous pression réduite et le résidu a été dissous dans du CH₂Cl₂ (20 mL). La phase organique a été lavée avec H₂O (3 x 10 mL), séchée sur MgSO₄, filtrée et le solvant a été éliminé sous pression réduite pour conduire de façon quantitative au composé **51a**. *R_{f}* (DCM/MeOH, 95/5) = 0.8; RMN ¹H (400 MHz, CDCl₃, δ): 7.95 (d, 1H, ⁴J 1.5, H⁵), 7.52 (d, 1H, ⁴J 1.5, H³), 5.35 (s, 2H, CH₂OMs), 3.98 (s, 2H, CH₂S), 3.82 (s, 2H, COCH₃), 3.77 (s, 2H, COCH₃), 3.14 (s, 3H, SCH₃).
**Composé 51b** : le composé **51b** a été préparé selon la même procédure que celle utilisée pour la synthèse de **51a**.
**Composés 52a-b** : les composés **52a-b** ont été préparés selon les mêmes procédures que celle utilisées respectivement pour la synthèse de **14b** et **14c.**
**Composés 53a-b** : les composés **53a-b** ont été préparés selon la même procédure que celle utilisée pour la synthèse de **46.**
**Composés 54a-b** : les composés **54a-b** ont été préparés selon la même procédure que celle utilisée pour la synthèse de **49.**
**Composés 55a-d** : les composés **55a-d** ont été préparés selon la même procédure que celle utilisée pour la synthèse de **50a.**
**Composés 56a-d** : les composés **56a-d** ont été préparés selon la même procédure que celle utilisée pour la synthèse de **51a**.
**Composé 57a** : le composé **57a** a été préparé selon la même procédure que celle utilisée pour la synthèse de **57b** en utilisant la pyridine **14a.**
**Composé** 57b : au dérivé bromé **14b** (103 mg, 0,35 mmol) a été ajouté du THF anhydre (1 mL) et la solution a été dégazée par trois cycles de congélation-décongélation. A cette solution ont été ajoutés du dérivé acétylénique (80 mg, 0,42 mmol) et de la TEA (0,24 mL, 1,75 mmol) et la solution a été à nouveau dégazée. A cette solution ont été ajoutés du Pd(dppf)Cl₂ (30 mg, 0,035 mmol) et du CuI (7 mg, 0,035 mmol). Cette nouvelle solution a été à nouveau dégazée trois fois puis a été agitée à 65 °C sous atmosphère inerte. L'avancement de la réaction a été suivi par CCM. Après 18 h la réaction était totale. Le mélange réactionnel a été refroidi à TA et le solvant a été éliminé sous pression réduite. Le produit brut a été purifié par chromatographie sur colonne de silice (DCM / MeOH 0 à 3 % par incréments de 0,1 %) pour obtenir une huile jaune correspondant au composé **57b** (122 mg, 87%). RMN ¹H (400 MHz, CDCl₃) δ: 7,98 (s I, 1H), 7,50 (s I, 1H), 7,45 (d, *J =* 8,9 Hz, 2H), 6,88 (d, *J* = 8,9 Hz, 2H), 4,80 (s, 2H), 4,65 (s, 2H), 4,09 (m, 1H), 3,99 (s I, 1H), 3,86 (m, 1H), 3,79 (s, 3H), 1,76 (d, J= 14,9 Hz, 3H), 1,26 (t, J= 6,9 Hz, 3H); RMN ¹³C (100 MHz, CDCl₃) δ: 168,9; 161,0 (d, *J* = 19 Hz), 158,8; 153,3 (d, *J* = 155 Hz), 133,8; 132,9 (d, *J* = 12 Hz), 128,1 (d, *J* = 22 Hz), 124,2; 115,1; 115,0; 95,7; 85,6; 65,2; 64,3; 61,3 (d, *J* = 5 Hz), 52,5; 16,5 (d, *J* = 4 Hz), 13,5 (d, *J* = 104 Hz); RMN ³¹P (162 MHz, CDCl₃) δ: +39,5. HRMS (ESI+) calculée pour C₂₀H₂₂NNaO₆P [M+Na]⁺, *m*/*z* 426,1082 trouvée: 426,1063. *R_{f}* = 0,44 (silice; DCM - MeOH 90:10).
**Composé 57c** : le composé **57c** a été préparé selon la même procédure que celle utilisée pour la synthèse de **57b** en utilisant la pyridine **14c.**
**Composé 59a** : une solution de dérivé acétylènique (0,864 g, 3,1 mmol) et de dérivé iodé **14a** (0,735 g, 2,5 mmol) dans un mélange de THF anhydre (20 mL) et de TEA (20 mL) a été dégazée sous agitation pendant 20 min. A cette solution ont été additionnés du bis-chlorure bis-triphénylphosphine de palladium (II) (22 mg, 0,031 mmol) et du CuI (12 mg, 0,063 mmol). La réaction a été agitée à TA pendant 12 h. L'avancement de la réaction a été suivi par CCM. Après cette période la réaction était totale. Les solvants ont été éliminés sous pression réduite. Au résidu a été additionnée une solution de chlorure d'ammonium saturée (50 mL) et le mélange a été extrait avec du DCM (2 x 25 mL). Les phases organiques ont été réunies, lavées par une solution de chlorure d'ammonium saturée (50 mL) puis avec une solution saturée de NaCl (2 x 50 mL) et ensuite séchées sur MgSO₄. Après filtration le solvant a été éliminé sous pression réduite et le résidu a été purifié par chromatographie sur colonne de silice (DCM / MeOH 98/2) pour conduire au composé **59a** sous forme d'un solide blanc (0,91 g, 82%). PtF : 143-144°C. RMN ¹H (500 MHz, CDCl₃) δ: 8,04 (s, 1H), 7,58 (s, 1H), 7,45 (d, *J* = 8,7 Hz, 2H), 6,86 (d, *J* = 8,7 Hz, 2H), 4,83 (d, *J* = 5,4 Hz, 2H), 4,75 (s, 1H), 4,01 (t, *J* = 6,1 Hz, 2H), 3,97 (s, 3H), 3,31 (td, J= 6,1; 6,1 Hz, 2H), 1,96 (m, *J=* 6,1 Hz, 2H), 1,41 (s, 9H). RMN ¹³C (125 MHz, CDCl₃) δ: 165,4; 160,8; 160,1; 156,2; 147,3; 134,1; 133,8; 125,8; 125,4; 114,9; 113,9; 95,9; 85,4; 66,1; 64,8; 53,2; 38,1; 29,7; 28,6. HRMS (ESI+) calculée pour C₂₄H₂₃N₂O₅ [M+H]⁺, *m*/*z* 441,2020, trouvée: 441,2021. *R_{f}* = 0,32 (silice, DCM - MeOH 96:4).
**Composé 59b** : au dérivé bromé **14b** (200 mg, 0,68 mmol) a été ajouté du THF anhydre (10 mL) et la solution a été dégazée par trois cycles de congélation-décongélation. A cette solution ont été ajoutés du dérivé acétylénique (260 mg, 0,75 mmol) et de la TEA (5 mL) et la solution a été à nouveau dégazée. A cette solution ont été ajoutés du Pd(PPh₃)₄ (79 mg, 0,068 mmol) et du CuI (13 mg, 0,068 mmol). Cette nouvelle solution a été à nouveau dégazée trois fois puis a été agitée à 65 °C sous atmosphère inerte. L'avancement de la réaction a été suivi par CCM. Après 1 h la réaction était totale. Le mélange réactionnel a été refroidi à TA et le solvant a été éliminé sous pression réduite. Le résidu a été dilué dans du DCM (25 mL) et lavé avec une solution aqueuse saturée de chlorure d'ammonium (25 mL) puis avec de l'eau (25 mL). La phase organique a été séchée sur MgSO₄, filtrée puis concentrée sous pression réduite pour conduire à un résidu qui a été purifié par chromatographie sur colonne de silice (DCM / MeOH 0 à 5 % par incréments de 1 %) pour obtenir une huile jaune correspondant au composé **59b** (220 mg, 66 %). HRMS (ESI+) calculée pour C₂₅H₃₄N₂O₅P [M+H]⁺, *m*/*z* 489,2149 trouvée: 489,2152. R*f* = 0,35 (silice, DCM - MeOH, 95:5).
**Composé 59c :** au dérivé bromé **14c** (142 mg, 0,4 mmol) a été ajouté du THF anhydre (10 mL) et la solution a été dégazée par trois cycles de congélation-décongélation. A cette solution ont été ajoutés du dérivé acétylénique (147 mg, 0,4 mmol) et de la TEA (5 mL) et la solution a été à nouveau dégazée. A cette solution ont été ajoutés du Pd(PPh₃)₄ (46 mg, 0,04 mmol) et CuI (7,6 mg, 0,04 mmol). Cette nouvelle solution a été à nouveau dégazée trois fois puis a été agitée à 65 °C sous atmosphère inerte. L'avancement de la réaction a été suivi par CCM. Après 1 h la réaction était totale. Le mélange réactionnel a été refroidi à TA et le solvant a été éliminé sous pression réduite. Le résidu a été dilué dans du DCM (25 mL) et lavé avec une solution aqueuse saturée de chlorure d'ammonium (25 mL) puis avec de l'eau (25 mL). La phase organique a été séchée sur MgSO₄, filtrée puis concentrée sous pression réduite pour conduire à un résidu qui a été purifié par chromatographie sur colonne de silice (DCM / MeOH 0 à 3 % par incréments de 0,5 %) pour obtenir une huile jaune correspondant au composé **59c** (154 mg, 70 %). RMN ¹H (400 MHz, CDCl₃) δ : 8,01 (dd, *J* = 6,4; 2,0 Hz, 1H), 7,89 (dd, *J* = 8,4; 12,4 Hz, 2H), 7,48 (t, *J* = 8,4 Hz, 1H), 7,40 (d, *J* = 8,8 Hz, 2H), 7,39 (td, *J* = 8,4; 4,2 Hz, 2H), 7,33 (d, *J* = 2,0 Hz, 1H), 6,81 (d, *J* = 8,8 Hz, 2H), 4,73 (s, 1H), 4,69 (s, 2H), 4,08 (qd, *J* = 5,6; 4,8 Hz, 2H), 3,97 (t, *J* = 6 Hz, 2H), 3,26 (m, 2H), 1,92 (q, *J* = 6 Hz, 2H), 1,37 (s, 9H, 1,31 (t, *J* = 5,6 Hz, 3H) ; RMN ¹³C (100 MHz, CDCl₃) δ : 160,3 (d, *J* = 18 Hz), 159,8; 156,0; 153,2 (d, *J =* 164 Hz); 133,7; 133,0 (d, *J* = 11 Hz); 132,6 (d, *J* = 5 Hz); 132,3 (d, *J* = 10 Hz); 129,6 (d, *J* = 138 Hz); 128,6 (d, *J* = 18 Hz); 128,5 (d, *J* = 9 Hz); 123,8 (d, *J* = 3 Hz); 114,7; 113,8; 96,0; 85,3 (d, *J* = 2 Hz); 79,3; 65,9; 63,8; 61,9 (d, *J* = 6 Hz); 37,9; 29,5; 28,4; 16,6; RMN ³¹P (162 MHz, CDCl₃) δ: +25,6. HRMS (ESI+) calculée pour C₃₀H₃₆N₂O₆P [M+H]⁺, *m*/*z* 551,2306 trouvée: 551,2305. R*f* = 0,24 (silice, DCM - MeOH, 95:5).
**Composé 60a** : A une solution d'alcool **59a** (195 mg, 0,44 mmol) dans du THF anhydre (7 mL), a été ajoutée goutte à goutte de la TEA (0,2 mL, 148 µmol) sous atmosphère inerte. A ce mélange refroidi à 4°C, a été ajoutée goutte à goutte du MsCl (67 µL, 0,84 mmol). L'avancement de la réaction a été suivi par CCM. Après 5 min, la réaction était totale. Le solvant a été éliminé sous pression réduite. Le résidu a été dissous dans du DCM (10 mL) et cette solution a été lavée avec de l'eau (2 x 10 mL). La phase organique a été séchée sur MgSO₄, filtrée et concentrée sous pression réduite pour conduire à une huile de couleur jaune-verte (240 mg, quantitatif). Le produit **60a** était suffisamment pur pour être utilisé dans la suite de la synthèse sans purification supplémentaire. LRMS (ESI+) calculée pour C₂₅H₃₁N₂O₈S [M+H]⁺, *m*/*z* 519,1801, trouvée: 519,13. R*f =* 0,6 (silice, DCM - MeOH 96:4). **Composés 60b-c:** les composés **60b-c** ont été préparés selon la même procédure que celle utilisée pour la synthèse de **60a.**
**Composés 61a-c** : les composés **61a-c** ont été préparés selon la même procédure que celle utilisée pour la synthèse de **61d**.
**Composé 61d** : à une solution de composé 1 (81 mg, 0,353 mmol) dans du THF anhydre (10 mL) ont été ajoutés le composé **17a** (234 mg, 0,706 mmol) en solution dans du MeCN anhydre (10 mL) puis le carbonate de potassium (195 mg, 1,413 mmol) en une seule fois. La réaction a été agitée à 60 °C pendant 1 nuit. L'avancement de la réaction a été suivi par UPLC-MS (gradient A). Après cette période, la réaction était totale. Le mélange réactionnel a été concentré sous pression réduite, dilué dans du DCM (50 mL), lavé avec de l'eau (25 mL x 2) puis de l'eau saturée en NaCl (25 mL). La phase organique a été séchée sur MgSO₄, filtrée et concentrée sous pression réduite. Le brut a été purifié par chromatographie sur colonne de silice en utilisant un gradient de solvant DCM/MeOH de 96/4 jusqu'à 90/10 pour conduire au composé **61d** (67 mg, 27%) sous forme de poudre blanche. HPLC gradient A - Rt = 2,91 min - [M+H]⁺, m/z 700,54 - HRMS (ESI+) calculée pour C₃₅H₅₀N₅O₁₀⁺ [M+H]+, m/z 700,3552 , trouvée : 700,3560 - RMN ¹H (400 MHz, CDCl₃) δ : 7,85 (s, 2 H), 7,69 (s, 1 H), 7,57 (s, 1 H), 4,00 (s, 3 H, Py-COOMe), 3,97 (s, 10 H), 3, (s, 3 H, OMs), 3,66 (s, 6 H), 3 (m, 20 H), 1,45 (s, 9 H) ; RMN ¹³C (100 MHz, CDCl₃) δ: 172,48; 172,39; 165,90; 161,48; 155,68; 151,13; 147,55; 147,43; 126,27; 123,70; 79,29; 63,56; 63,40; 57,15; 54,84; 54,66; 54,35; 52,88; 51,84; 51,80; 50,09; 49,50; 34,07; 33,93; 30,11; 30,06; 29,68; 28,56.
**Composés 61e-s** : les composés **61e-s** ont été préparés selon la même procédure que celle utilisée pour la synthèse de **61d.**
**Composés 62a-c** : les composés **62a-c** ont été préparés selon la même procédure que celle utilisée pour la synthèse de **62d**.
**Composés 62d:** dans un ballon de 25 mL le composé **61d** (67 mg, 0,096 mmol) a été solubilisé dans du DCM (500 µL) pour donner une solution incolore. Au mélange réactionnel a été ajouté du TFA (500 µL, 6,53 mmol) en une seule fois. La réaction a été agitée à TA pendant 30 min. L'avancement de la réaction a été suivi par UPLC-MS (gradient A). Après cette période, la réaction était totale. Le mélange réactionnel a été concentré sous pression réduite pour conduire à une poudre blanche (57 mg) qui a été utilisée dans la suite de la procédure. Dans un ballon de 100 mL le composé **60a** (76 mg, 0,147 mmol) et la poudre blanche obtenue précédemment à partir de deux lots réunis (87,4 mg, 0,122 mmol) ont été solubilisés dans du MeCN anhydre (30 mL) pour donner une solution incolore. Au mélange réactionnel a été ajouté le carbonate de potassium (16,92 mg, 0,122 mmol) en une seule fois. La réaction a été agitée à 65 °C pendant 1 nuit. L'avancement de la réaction a été suivi par UPLC-MS (gradient A), après cette période, la réaction était totale. Le mélange réactionnel a été concentré sous pression réduite, dilué dans du DCM (50 mL), lavé avec de l'eau (2 x 40 mL). La phase organique a été séchée sur MgSO₄, filtrée et concentrée sous pression réduite pour conduire au composé **62d** (10,8 mg, 10,6 µmol, 9%) sous forme de poudre blanche. HPLC gradient A - Rt = 2,91 min - [M+H]⁺, m/z 1022,65 - HRMS (ESI+) calculée pour C₅₄H₆₇N₇O₁₃Ag⁺ [M+Ag]⁺, m/z 1128,3842 , trouvée : 1128,3843.
**Composés 62e-s** : les composés **62e-s** ont été préparés selon la même procédure que celle utilisée pour la synthèse de **62d.**
**Composés 64a-c** : les composés **64a-c** ont été préparés selon la même procédure que celle utilisée pour la synthèse de **64d.**
**Composés 64d** : dans un ballon de 50 mL le composé **62d** (10,8 mg, 10,57 µmol) a été solubilisé dans de l'eau (4 mL) pour donner une suspension incolore. Au mélange réactionnel a été ajouté LiOH (1,291 mg, 0,053 mmol) en une seule fois. La réaction a été agitée à TA pendant 1 h. L'avancement de la réaction a été suivi par UPLC-MS (gradient A). Après cette période, la déprotection était totale. Le pH du mélange réactionnel a été ajusté a 7 avec du HCl 1 M. Au mélange réactionnel (composé **63d)** a été ajouté du chlorure d'europium hexahydrate (5,81 mg, 15,85 µmol) en une seule fois. La réaction a été agitée à température ambiante pendant 1 h, après cette période, la réaction était totale. Le mélange réactionnel a été directement purifié par HPLC préparative (gradient B) et a conduit au composé **64d** (6,32 mg, 5,74 µmol, 54%) sous forme de poudre blanche. HPLC gradient A - Rt = 2,62 min - [M-2H]⁺, m/z 1102,62 - HRMS (ESI+) calculée pour C₄₉H₅₅EuNO₁₃⁺ [M-2H]⁺, m/z 1100,3051, trouvée : 1100,3064.
**Composés 64e-s** : les composés **64e-s** ont été préparés selon la même procédure que celle utilisée pour la synthèse de **64d.**
**Complexe 65d-E₂** : dans un ballon de 25 mL le composé **64d** (6,32 mg, 5,74 µmol) a été solubilisé dans du DMSO anhydre (1 mL) pour donner une solution incolore. Au mélange réactionnel a été ajouté l'acide 3-amino-1-propanesulfonique (3,29 mg, 22,96 µmol), la DIPEA (4 µL, 23 µmol) puis le HATU (6,75 mg, 17,2 µmol) en une seule fois. La réaction a été agitée à TA pendant 15 min. L'avancement de la réaction a été suivi par UPLC-MS (gradient C), après cette période, la réaction était totale. Le mélange réactionnel a été directement purifié par HPLC préparative (gradient D) et a conduit au complexe soluble **65d-E₂** (5,87 mg, 4,37 µmol, 76%) sous forme de poudre blanche. HPLC gradient C - Rt = 2,19 min - [M-2H]⁺, m/z 1345,26 - HRMS (ESI+) calculée pour C₅₅H₇₀EuN₉O₁₇S₂²⁺ [M-H]²⁺, m/z 672,6768 , trouvée : 672,6769.
**Complexe 66d-E₂** : dans un ballon de 25 mL le complexe **65d-E₂** (5,64 mg, 4,2 µmol) a été solubilisé dans du TFA (200 µL) pour donner une solution jaune. La réaction a été agitée à TA pendant 30 min. L'avancement de la réaction a été suivi par UPLC-MS (gradient C). Après cette période, la déprotection était totale. Le mélange réactionnel a été concentré sous pression réduite puis purifié par HPLC préparative (gradient E) et a conduit au complexe **66d-E₂** (2,16 µmol, 51%) sous forme de poudre blanche. HPLC gradient C - Rt = 1,36 min - [M-2H]⁺, m/z 1243,45 - HRMS (ESI+) calculée pour C₅₀H₆₂EuN₉O₁₅S₂²⁺ [M-H]²⁺, m/z 622,6506 , trouvée : 622,6503.

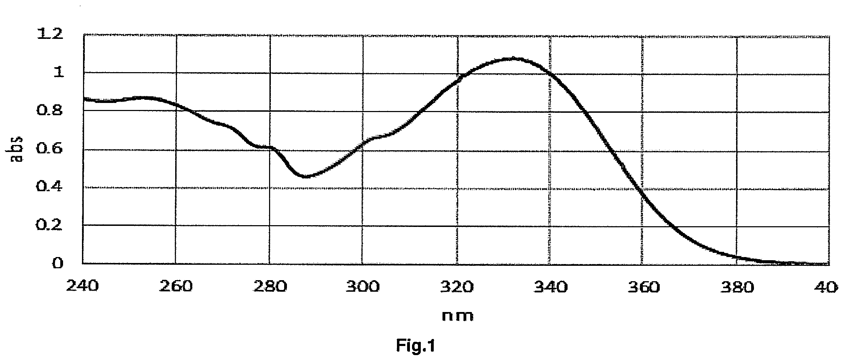

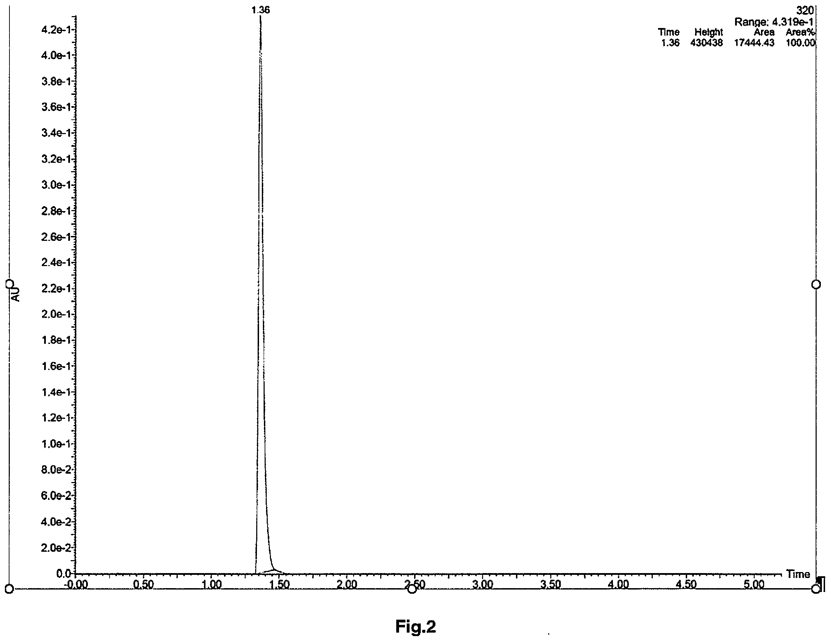

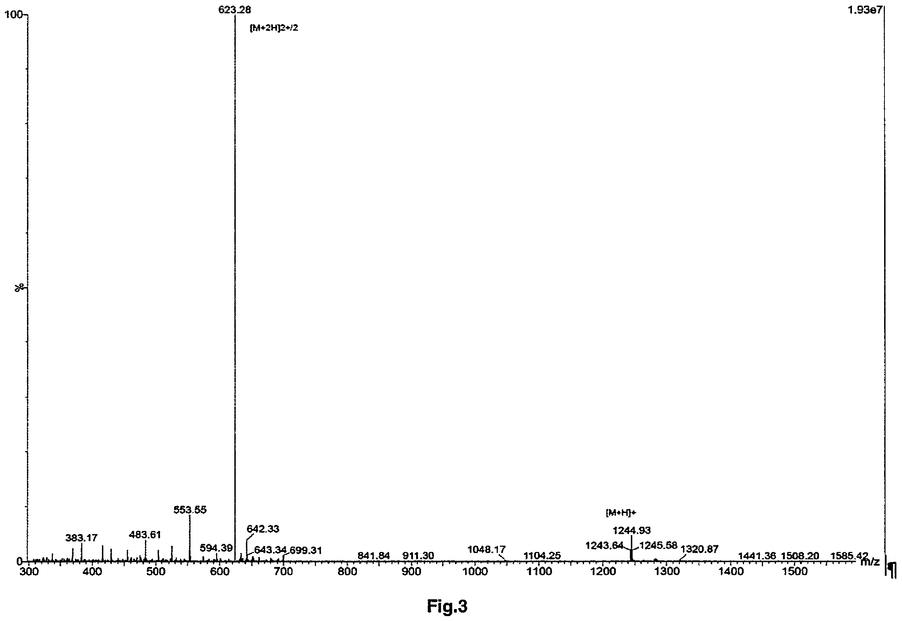

Le spectre UV, le chromatogramme et le spectre de masse du complexe **66d-E2** sont respectivement représentés aux figures 1 à 3.

Nous avons déterminé les propriétés photophysiques du complexe **66d-E2** et des complexes triantenne (TACN-Phos-triantenne et TACN-Carbo-triantenne), synthétisés comme décrit dans la demande WO 2014/111661, et dont la structure est représentée ci-dessous.

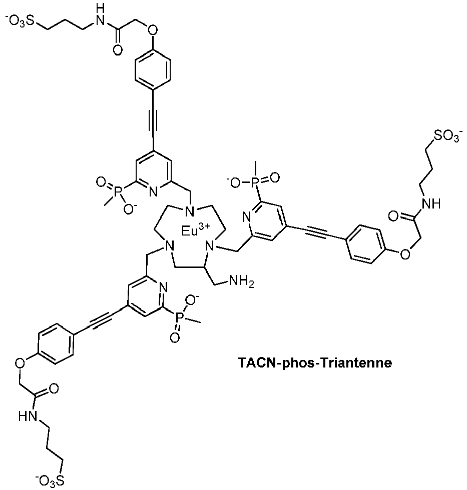

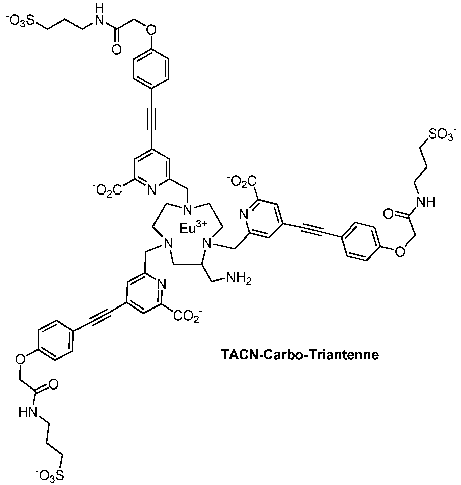

Comme nous pouvons le constater à la lecture du tableau ci-après, la brillance du complexe **66d-E2** est moins élevée que celle des complexes **TACN-Phos-triantenne** et **TACN-Carbo-triantenne** comprenant trois antennes.

| Complexe | Absorption max (nm) | Brillance | Durée de vie (ms) |
|---|---|---|---|
| 66d-E2 | 334 | 4800 | 0,93 |
| TACN-Phos-triantenne | 328 | 15000 | 1,04 |
| TACN-Carbo-triantenne | 337 | 11400 | 0,8 |

L'efficacité des complexes **66d-E2** et **TACN-Phos-triantenne** a par ailleurs été testée selon le protocole suivant. Chacun des complexes a été fonctionnalisé sous forme d'ester de NHS (N-hydroxysuccinimide) en utilisant les techniques classiques connues de l'homme du métier. Un lot d'anticorps anti GST (anti-Glutathione-S-transférase) a été marqué en utilisant soit le complexe **66dE2-NHS** soit le complexe **TACN-Phos triantenne-NHS.** Le nombre moyen de complexe fixé sur les anticorps est de 7, que ce soit avec le complexe TACN-Phos triantenne (AntiGST-TACN-Phos-triantenne) ou avec le complexe **66d-E2** (AntiGST-66d-E2).

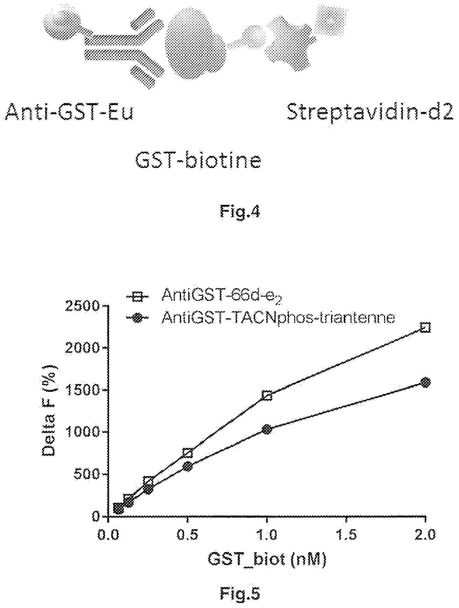

Les anticorps anti-GST marqués avec le complexe d'europium **66d-E₂ (AntiGST-66d-E2)** ou avec le complexe d'europium **TACN-Phos-triantenne (AntiGST-TACN Phos-triantenne)** ont été utilisés dans un immunoessai (représenté sur la figure 4) basé sur la fluorescence en temps-résolu pour détecter la protéine Glutathione-S-transferase-biotine (GST-biotine). Une gamme de concentrations de GST-biotine diluée dans du tampon phosphate contenant de la BSA a été mesurée en présence d'une concentration définie **d'anti-GST-66d-E₂** ou **d'antiGST-TACN Phos-triantenne** et de 5 nM de streptavidine-d2 (Cisbio Bioassays prod no 610SADLB) dans une plaque 384 puits en triplicate et lue en mode HTRF sur un lecteur de fluorescence de type Pherastar FS (BMG-labtech) après 2 h d'incubation. Les résultats sont reportés dans la figure 5. Avec les deux anticorps marqués, la GST-biotine est détectée. De façon surprenante et contre toute attente, la performance de l'immunoessai utilisant l'anticorps **AntiGST-66d-E2,** est supérieure à celle de **l'AntiGST-TACN phos-triantenne** c'est-à-dire que la sonde fluorescente mono-antenne présente une capacité de FRET supérieure à celle qui possède trois antennes, et ce malgré une brillance trois fois moins importante.

Pour confirmer ce résultat, un autre immunoessai a été réalisé en utilisant un anticorps qui reconnait l'adénosine monophosphate cyclique (AMPc). Pour cela l'anticorps anti-AMPc a été marqué avec le complexe **66d-E2-NHS** conduisant au conjugué **anti-AMPc-66d-E2** portant en moyenne 6,5 complexes/anticorps. Le même lot d'anticorps a été marqué avec le complexe **TACN-Carbo-triantenne NHS** conduisant au conjugué **anti-AMPc-TACN-Carbo-triantenne** portant en moyenne 6,6 complexes /anticorps.

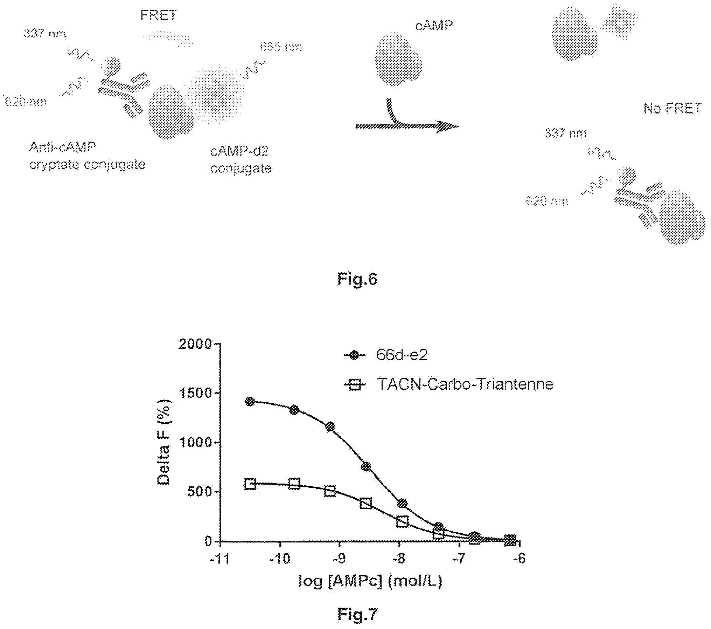

Le principe de l'essai est décrit dans la figure 6. Les deux conjugués ont été utilisés dans un essai par compétition en utilisant la fluorescence en temps-résolu comme technique de détection. L'anti-AMPc marqué avec un complexe d'europium reconnait l'AMPc conjuguée avec la fluorophore d2 (cAMP, Gs-dynamic kit, Cisbio Bioassays (ref 621M4PEC)) en donnant un signal de fluorescence en temps résolu. Une gamme de concentration d'AMPc permet de déplacer l'AMPc-d2 ce qui conduit à une diminution progressive du signal.

Les deux conjugués **anti-AMPc-66d-E2** et **anti-AMPc-TACN-Carbo-triantenne** ont été utilisés à une concentration bien définie en présence d'AMPc-d2 dans un tampon phosphate en présence d'albumine de sérum bovin (BSA) en faisant une gamme de concentrations de cAMP dans une plaque 384 puits en triplicate et lue en mode HTRF sur un lecteur de plaque de type Pherastar FS (BMG-labtech) après 1 h d'incubation. La réponse d'inhibition du signal de FRET est représentée dans la figure 7. Dans ce second exemple, et encore de façon surprenante, les performances du conjugué **anti-AMPc-66d-E2** sont supérieures à celles du conjugué **anti-AMPc-TACN-Carbo-triantenne,** c'est-à-dire que la sonde fluorescente mono-antenne présente une capacité de FRET supérieure à celle qui possède trois antennes, et ce malgré une brillance deux fois moins importante.

Les agents complexants constitués d'un macrocycle TACN, portant un ou deux chromophores de type phényléthynylpyridine, forment des complexes stables avec les lanthanides, et peuvent être utilisés pour produire des conjugués fluorescents de molécules d'intérêt. Les complexes de lanthanide selon l'invention présentent d'excellentes propriétés photophysiques, en particulier en ce qui concerne leur rendement quantique, la durée de vie de leur luminescence et leur spectre d'excitation qui est très bien adapté à une excitation laser à environ 337 nm. De plus la répartition des bandes de leurs spectres d'émission est centrée autour de 620 nm conférant ainsi aux complexes des propriétés exceptionnelles et très favorables dans une utilisation de FRET avec des accepteurs de type cyanine ou allophycocyanine (telle que la XL665 commercialisée par Cisbio Bioassays). Du fait de la grande stabilité de ces complexes dans les milieux biologiques contenant la plupart des cations divalents (Ca²⁺, Mg²⁺...) ou de l'EDTA, leur luminescence reste excellente.

## Revendications

1. Agent complexant de formule (I) : 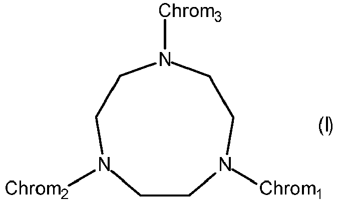 dans laquelle :
- Chrom₁, Chrom₂ et Chrom₃ représentent indépendamment l'un de l'autre un groupe de formule : 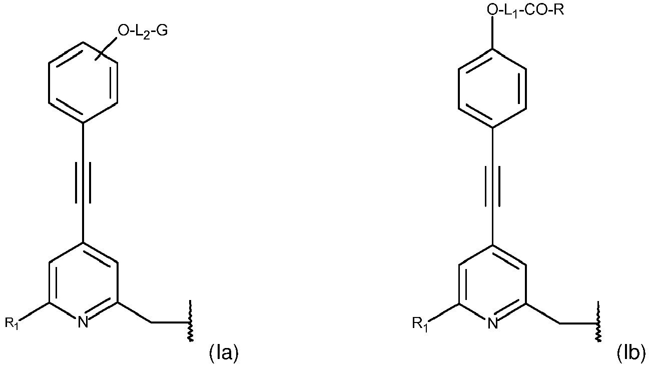 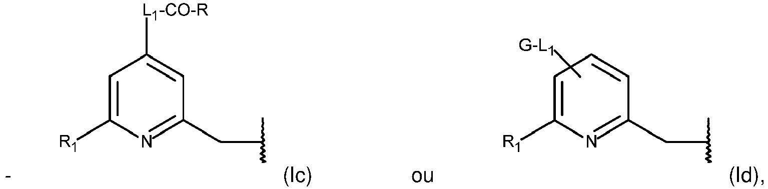 étant entendu que le composé de formule (I) comprend (i) un ou deux groupes choisis parmi les groupes (la) et (Ib) et (ii) au moins un groupe L₁-CO-R ;
- R est un groupe ―OR₂ ou -NH-E ;
- R₁ est un groupe ―CO₂H ou ―P(O)(OH)R₃ ;
- R₂ est H ou un (C₁-C₄)alkyle ;
- R₃ est un (C₁-C₄)alkyle, de préférence un méthyle ; un phényle éventuellement substitué par un groupe ―SO₃⁻, ce dernier étant de préférence en position méta ou para ; ou un benzyle ;
- L₁ est une liaison directe ; un groupe ―(CH₂)ᵣ- éventuellement interrompu par au moins un atome choisi parmi un atome d'oxygène, un atome d'azote et un atome de soufre ; un groupe -CH=CH- ; un groupe ―CH=CH-CH₂- ; un groupe ―CH₂-CH=CH- ; ou un groupe PEG ;
- L2 est un groupe de liaison divalent ;
- G est un groupe réactif choisi parmi une amine éventuellement protégée sous forme -NHBoc, un ester de succinimidyle, un haloacétamide, une hydrazine, un isothiocyanate, un groupe maléimide, ou un acide carboxylique éventuellement protégé sous la forme d'un groupe ―CO₂Me ou -CO₂tBu. ;
- E est un groupe ―CH₂-(CH₂)ₛ-CH₂-SO₃⁻ ou ―(CH₂)ₛ-N⁺Alk₁Alk₂Alk₃, ou une sulfobétaïne, ladite sulfobétaïne ayant l'une des formules ci-après : 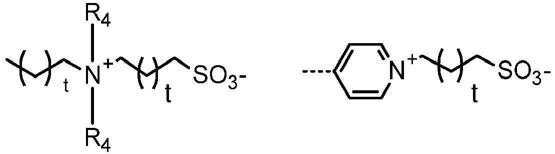 avec R₄ qui représente un (C₁-C₆)alkyle, et t qui est égal à 1, 2, 3, 4, 5 ou 6,
- r est un entier allant de 1 à 6, de préférence de 1 à 3 ;
- s est 0, 1 ou 2 ;
- Alk₁, Alk₂, Alk₃, qui peuvent être identiques ou différents, représentent un (C₁-C₆)alkyle.

2. Agent complexant selon la revendication 1, dans lequel Chrom₁ est un groupe de formule (la) et Chrom₂ et Chrom₃ sont chacun un groupe de formule (Ic), identique ou différent.

3. Agent complexant selon la revendication 2, dans lequel Chrom₂ et Chrom₃ sont identiques.

4. Agent complexant selon la revendication 1, dans lequel Chrom₁ et Chrom₂ sont chacun un groupe de formule (Ib), identique ou différent, et Chrom₃ est un groupe de formule (Id).

5. Agent complexant selon la revendication 4, dans lequel Chrom₁ et Chrom₂ sont identiques.

6. Agent complexant selon l'une quelconque des revendications précédentes, dans lequel R₁ est un groupe ―CO₂H ou -P(O)(OH)R₃ dans lequel R₃ est un (C₁-C₄)alkyle ou un phényle.

7. Agent complexant selon l'une quelconque des revendications précédentes, dans lequel L₁ est est une liaison directe ; un groupe ―(CH₂)ᵣ- éventuellement interrompu par au moins un atome choisi parmi un atome d'oxygène et un atome de soufre, et r = 2 ou 3 ; un groupe -CH=CH- ; un groupe - CH=CH-CH₂- ; ou un groupe ―CH₂-CH=CH.

8. Agent complexant selon l'une quelconque des revendications précédentes, dans lequel E est un groupe ―CH₂-(CH₂)ₛ-CH₂-SO₃⁻ avec s = 0 ou 1 ; ―(CH₂)ₛ-N⁺Alk₁Alk₂Alk₃ avec Alk₁, Alk₂ Alk₃, identiques ou différents, représentant un (C₁-C₄)alkyle et s = 0 ou 1 ; ou un groupe de formule : 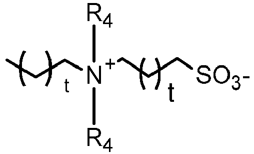 dans laquelle R₄ est un (C₁-C4)alkyle et t est 1 ou 2.

9. Agent complexant selon l'une quelconque des revendications précédentes, dans lequel L₂ est choisi parmi :
- une liaison directe;
- un groupe alkylène linéaire ou ramifié en C₁-C₂₀, contenant éventuellement une ou plusieurs doubles ou triples liaisons ;
- un groupe cycloalkylène en C₅-C₈ ; un groupe arylène en C₆-C₁₄; lesdits groupes alkylène, cycloalkylène ou arylène contenant éventuellement un ou plusieurs hétéroatomes, tels que l'oxygène, l'azote, le soufre, le phosphore, ou un ou plusieurs groupe(s) carbamoyle ou carboxamido, et lesdits groupes alkylène, cycloalkylène ou arylène étant éventuellement substitués par 1 à 5 groupes alkyle en C₁-C₈, aryle en C₆-C₁₄, sulfonate ou oxo.
- un groupe choisi parmi les groupes divalents de formules suivantes : —(CH2)ₙ— ;—(CH₂)ₙ—O—(CH₂)ₘ—O—(CH₂)ₚ— ; 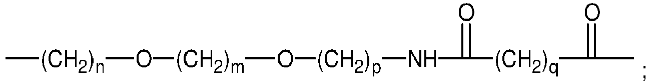 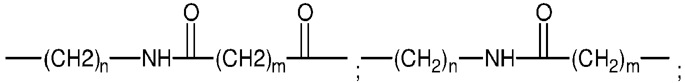 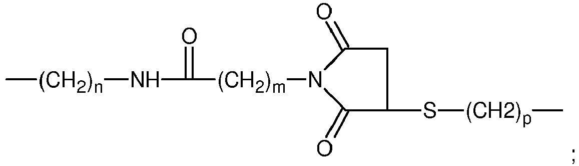 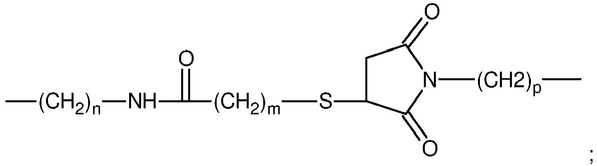 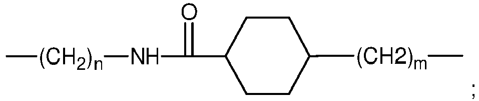 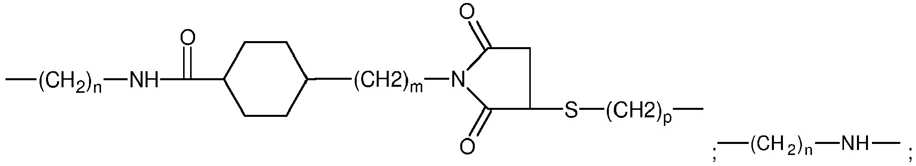 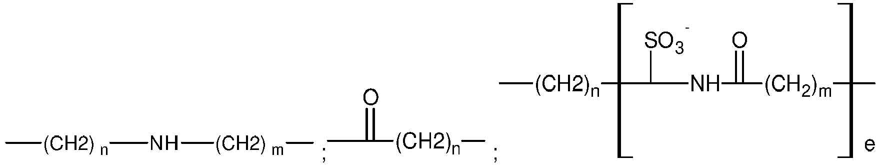 dans lesquelles n, m, p, q sont des nombres entiers de 1 à 16, de préférence de 1 à 5 et e est un nombre entier allant de 1 à 6, de préférence de 1 à 4.

10. Agent complexant selon l'une quelconque des revendications précédentes, dans lequel le groupe ―L₂-G est constitué d'un groupement réactif G tel que défini à la revendication 1, et d'un bras d'espacement L₂ constitué d'une chaîne alkylène comprenant de 1 à 5 atomes de carbone ou d'un groupe choisi parmi les groupes de formule : 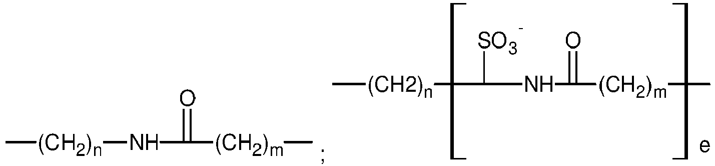 où n, m sont des nombres entiers de 1 à 16, de préférence de 1 à 5 et e est un nombre entier allant de 1 à 6, de préférence de 1 à 4, le groupe G étant lié à l'une ou l'autre extrémité de ces groupes divalents.

11. Complexe de lanthanide comprenant un agent complexant selon l'une quelconque des revendications précédentes et un lanthanide.

12. Complexe de lanthanide selon la revendication 11, **caractérisé en ce que** le lanthanide est choisi parmi : Eu³⁺, Sm³⁺, Tb³⁺, Gd³⁺, Dy³⁺, Nd³⁺, Er³⁺.

13. Complexe de lanthanide selon la revendication 12, **caractérisé en ce que** le lanthanide est est Eu³⁺ ou Tb³⁺, de préférence Eu³⁺.

14. Conjugué obtenu par réaction entre (i) un complexe de lanthanide selon l'une quelconque des revendications 11 à 13 comprenant un groupe G et (ii) une molécule d'intérêt comprenant un groupe fonctionnel, ledit groupe fonctionnel formant une liaison covalente avec l'un des atomes du groupe G, ladite molécule d'intérêt étant choisie parmi :un acide aminé, un peptide, une protéine, un anticorps, un sucre, une chaîne glucidique, un nucléoside, un nucléotide, un oligonucléotide ou un substrat d'enzyme.

15. Conjugué selon la revendication 14, dans lequel le substrat d'enzyme est la benzylguanine, la benzylcytosine, un chloroalcane ou le coenzyme A.

## Patentansprüche

1. Komplexbildner der Formel (I): 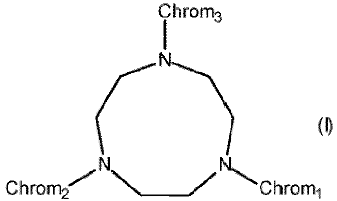 wobei:
- Chrom_{1,} Chrom₂ und Chrom₃ unabhängig voneinander eine Gruppe der Formel: 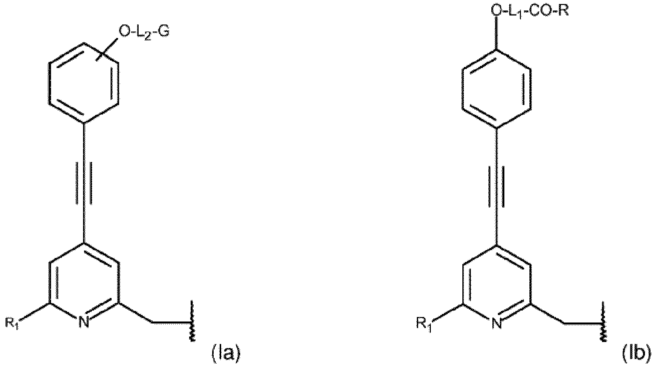 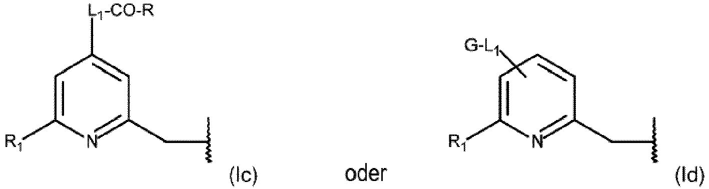 repräsentieren, wobei es sich versteht, dass die Verbindung der Formel (I) (i) eine oder zwei Gruppen, die aus den Gruppen (la) und (Ib) ausgewählt sind, und (ii) mindestens eine Gruppe L₁-CO-R umfasst,
- R eine Gruppe -OR₂- oder -NH-E ist,
- R₁ eine Gruppe -CO₂H oder -P(O)(OH)R₃ ist,
- R₂ H oder ein (C₁-C₄)-Alkyl ist,
- R₃ ein (C₁-C₄)-Alkyl, bevorzugt ein Methy; ein Phenyl, das gegebenenfalls durch eine Gruppe -SO₃⁻ substituiert ist, wobei diese Letztere bevorzugt in meta- oder para-Position vorliegt; oder ein Benzyl ist,
- L₁ eine Direktbindung, eine Gruppe -(CH₂)ᵣ-, die gegebenenfalls durch mindestens ein Atom unterbrochen ist, das aus einem Sauerstoffatom, einem Stickstoffatom und einem Schwefelatom ausgewählt ist; eine Gruppe -CH=CH-; eine Gruppe - CH=CH-CH₂-; eine Gruppe -CH₂-CH=CH-; oder eine PEG-Gruppe ist,
- L₂ eine divalente Bindungsgruppe ist,
- G eine Reaktionsgruppe ist, die aus einem Amin, das gegebenenfalls in Form von -NHBoc geschützt ist, einem Succinimidylester, einem Halogenacetamid, einem Hydrazin, einem Isothiocyanat, einer Maleimidgruppe oder einer Carbonsäure, die gegebenenfalls in Form einer Gruppe ―CO₂Me oder -CO₂tBu geschützt ist, ausgewählt ist,
- E eine Gruppe -CH₂-(CH₂)ₛ-CH₂-SO₃⁻ oder -(CH₂)ₛ-N⁺Alk₁Alk₂Alk₃ oder ein Sulfobetain ist, wobei das Sulfobetain eine der folgenden Formeln aufweist: 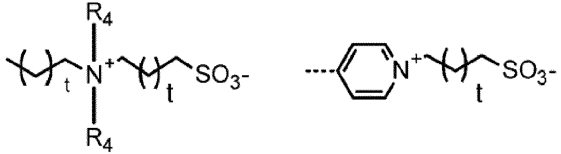 wobei R₄ ein (C₁-C₆)-Alkyl repräsentiert und t gleich 1, 2, 3, 4, 5 oder 6 ist,
- r eine ganze Zahl von 1 bis 6, bevorzugt 1 bis 3, ist,
- s 0, 1 oder 2 ist,
- Alk₁, Alk₂, Alk₃, die identisch oder verschieden sein können, ein (C₁-C₆)-Alkyl repräsentieren.

2. Komplexbildner nach Anspruch 1, wobei Chrom₁ eine Gruppe der Formel (la) ist und Chrom₂ und Chrom₃ jeweils eine Gruppe der Formel (Ic) sind, die identisch oder verschieden sind.

3. Komplexbildner nach Anspruch 2, wobei Chrom₂ und Chrom₃ identisch sind.

4. Komplexbildner nach Anspruch 1, wobei Chrom₁ und Chrom₂ jeweils eine Gruppe der Formel (Ib) sind, die identisch oder verschieden sind, und Chrom₃ eine Gruppe der Formel (Id) ist.

5. Komplexbildner nach Anspruch 4, wobei Chrom₁ und Chrom₂ identisch sind.

6. Komplexbildner nach einem der vorhergehenden Ansprüche, wobei R₁ eine Gruppe -CO₂H oder -P(O)(OH)R₃ ist, wobei R₃ ein (C₁-C₄)-Alkyl oder ein Phenyl ist.

7. Komplexbildner nach einem der vorhergehenden Ansprüche, wobei L₁ eine Direktbindung; eine Gruppe -(CH₂)ᵣ-, die gegebenenfalls durch mindestens ein Atom unterbrochen ist, das aus einem Sauerstoffatom und einem Schwefelatom ausgewählt ist, und wobei r = 2 oder 3; eine Gruppe -CH=CH-; eine Gruppe -CH=CH-CH₂-; oder eine Gruppe -CH₂-CH=CH ist.

8. Komplexbildner nach einem der vorhergehenden Ansprüche, wobei E eine Gruppe -CH₂-(CH₂)ₛ-CH₂-SO₃⁻, wobei s = 0 oder 1; -(CH₂)ₛ-N⁺Alk₁Alk₂Alk₃, wobei Alk₁, Alk₂ Alk₃, die identisch oder verschieden sind, ein (C₁-C₄)-Alkyl repräsentieren und s = 0 oder 1; oder eine Gruppe der Formel: 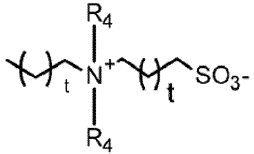 ist, wobei R₄ ein (C₁-C₄)-Alkyl ist und t 1 oder 2 ist.

9. Komplexbildner nach einem der vorhergehenden Ansprüche, wobei L₂ ausgewählt ist aus:
- einer Direktbindung,
- einer linearen oder verzweigten C₁-C₂₀-Alkylengruppe, die gegebenenfalls eine oder mehrere Doppel- oder Dreifachbindungen enthält,
- einer C₅-C₈-Cycloalkylengruppe, einer C₆-C₁₄-Arylengruppe,
wobei die Alkylen-, Cycloalkylen- oder Arylengruppen gegebenenfalls ein oder mehrere Heteroatome, wie etwa Sauerstoff, Stickstoff, Schwefel, Phosphor, oder eine oder mehrere Carbamoyl- oder Carboxamidogruppe(n) enthalten und die Alkylen-, Cycloalkylen- oder Arylengruppen gegebenenfalls durch 1 bis 5 C₁-C₈-Alkyl-, C₆-C₁₄-Aryl-, Sulfonat- oder Oxogruppen substituiert sind,
- einer Gruppe, die ausgewählt ist aus den divalenten Gruppen der folgenden Formeln: —(CH₂)ₙ—;—(CH₂)ₘ—O—(CH₂)ₚ; 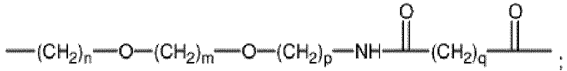 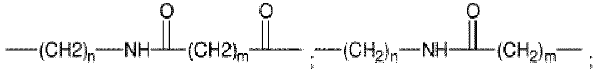 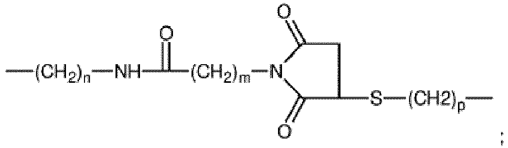 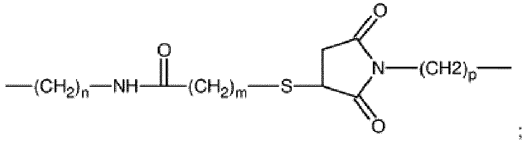 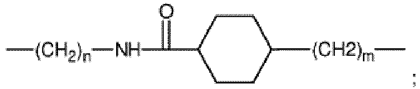 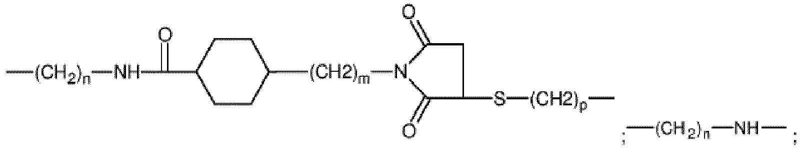 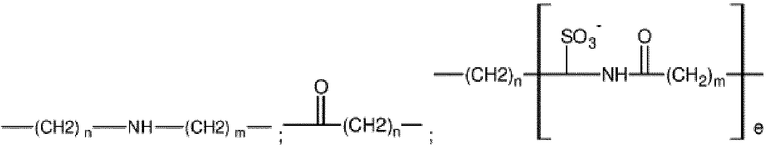 wobei n, m, p, q ganze Zahlen von 1 bis 16, bevorzugt 1 bis 5, sind und e eine ganze Zahl von 1 bis 6, bevorzugt 1 bis 4, ist.

10. Komplexbildner nach einem der vorhergehenden Ansprüche, wobei die Gruppe -L₂-G aus einer reaktiven Gruppierung G, wie in Anspruch 1 definiert, und einem Abstandsarm L₂ besteht, der aus einer Alkylenkette, die 1 bis 5 Kohlenstoffatome umfasst, oder einer Gruppe besteht, die ausgewählt ist aus den Gruppen der Formel: 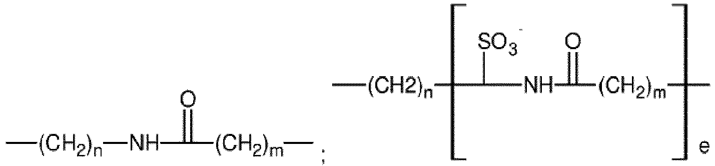 wobei n, m ganze Zahlen von 1 bis 16, bevorzugt 1 bis 5, sind und e eine ganze Zahl von 1 bis 6, bevorzugt 1 bis 4, ist, wobei die Gruppe G an das eine oder das andere Ende dieser divalenten Gruppen gebunden ist.

11. Lanthanoidkomplex, umfassend einen Komplexbildner nach einem der vorhergehenden Ansprüche und ein Lanthanoid.

12. Lanthanoidkomplex nach Anspruch 11, **dadurch gekennzeichnet, dass** das Lanthanoid ausgewählt ist aus: Eu³⁺, Sm³⁺, Tb³⁺, Gd³⁺, Dy³⁺, Nd³⁺, Er³⁺.

13. Lanthanoidkomplex nach Anspruch 12, **dadurch gekennzeichnet, dass** das Lanthanoid Eu³⁺ oder Tb³⁺, bevorzugt Eu³⁺, ist.

14. Konjugat, das durch Reaktion zwischen (i) einem Lanthanoidkomplex nach einem der Ansprüche 11 bis 13, umfassend eine Gruppe G, und (ii) einem interessierenden Molekül, umfassend eine funktionelle Gruppe, erlangt wird, wobei die funktionelle Gruppe eine kovalente Bindung mit einem der Atome der Gruppe G bildet, wobei das interessierende Molekül ausgewählt ist aus: einer Aminosäure, einem Peptid, einem Protein, einem Antikörper, einem Zucker, einer Kohlenhydratkette, einem Nukleosid, einem Nukleotid, einem Oligonukleotid oder einem Enzymsubstrat.

15. Konjugat nach Anspruch 14, wobei das Enzymsubstrat Benzylguanin, Benzylcytosin, ein Chloralkan oder Coenzym A ist.

## Claims

1. A complexing agent of formula (I): 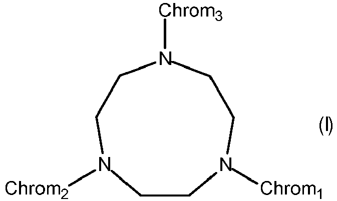 wherein:
- Chrom₁, Chrom₂ and Chrom₃, independently of one another, represent a group of formula: 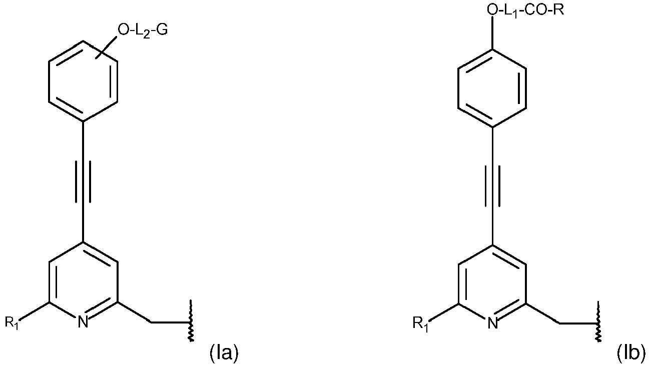 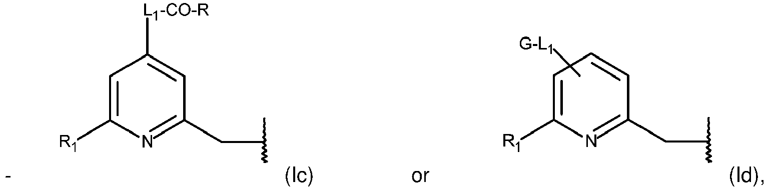 provided that the compound of formula (I) comprises (i) one or two groups selected from the groups (Ia) and (Ib) and (ii) at least one L₁-CO-R group;
- R is a ―OR₂ or -NH-E group;
- R₁ is a ―CO₂H or ―P(O)(OH)R₃ group;
- R₂ is H or a (C₁-C₄)alkyl;
- R₃ is a (C₁-C₄)alkyl, preferably methyl; phenyl optionally substituted by a ―SO₃⁻ group, the latter preferably in the meta or para position; or benzyl;
- L₁ is a direct bond; a ―(CH₂)ᵣ― group optionally interrupted by at least one atom selected from an oxygen atom, a nitrogen atom and a sulphur atom; a -CH=CH- group; a -CH=CH-CH₂- group; a ―CH₂-CH=CH- group; or a PEG group;
- L₂ is a divalent linking group;
- G is a reactive group selected from an amine optionally protected in -NHBoc form, a succinimidyl ester, a haloacetamide, a hydrazine, an isothiocyanate, a maleimide group, or a carboxylic acid optionally protected as a ―CO₂Me, -CO₂tBu group.;
E is a ―CH₂-(CH₂)ₛ-CH₂-SO₃⁻ or ―(CH₂)ₛ-N⁺Alk₁Alk₂Alk₃ group, or a sulfobetaine, wherein said sulfobetaine has one of the following formulae: 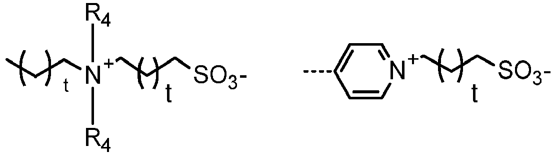 with R₄ which represents a (C₁-C₆)alkyl, and t which is equal to 1, 2, 3, 4, 5 or 6;
- r is an integer from 1 to 6, preferably from 1 to 3;
- s is 0, 1 or 2;
- Alk₁, Alk₂, Alk₃, which may be identical or different, represent a (C₁-C₆)alkyl.

2. The complexing agent according to claim 1, wherein Chrom₁ is a group of formula (Ia) and Chrom₂ and Chrom₃ are each a group of formula (Ic), identical or different.

3. The complexing agent according to claim 2, wherein Chrom₂ and Chrom₃ are identical.

4. The complexing agent according to claim 1, wherein Chrom₁ and Chrom₂ are each a group of formula (Ib), identical or different, and Chrom₃ is a group of formula (Id).

5. The complexing agent according to claim 4, wherein Chrom₁ and Chrom₂ are identical.

6. The complexing agent according to any one of the preceding claims, wherein R₁ is a ― CO₂H or -P(O)(OH)R₃ group wherein R₃ is a (C₁-C₄)alkyl or phenyl.

7. The complexing agent according to any one of the preceding claims, wherein L₁ is a direct bond; a ―(CH₂)ᵣ― group optionally interrupted by at least one atom selected from an oxygen atom and a sulphur atom, and r = 2 or 3; a -CH=CH- group; a ―CH=CH-CH₂- group; or a ―CH₂-CH=CH-group.

8. The complexing agent according to any one of the preceding claims, wherein E is a ―CH₂-(CH₂)ₛ-CH₂-SO₃⁻ group with s = 0 or 1; ―(CH₂)ₛ-N⁺Alk₁Alk₂Alk₃ with Alk₁, Alk₂ Alk₃, identical or different, representing (C₁-C₄)alkyl and s = 0 or 1; or a group of formula: 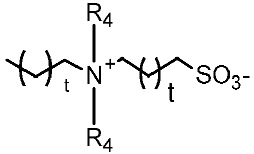 wherein R₄ is a (C₁-C₄)alkyl and t is 1 or 2.

9. The complexing agent according to any one of the preceding claims, wherein L₂ is selected from:
- a direct bond;
- a linear or branched C₁-C₂₀ alkylene group, optionally containing one or more double or triple bonds;
- a C₅-C₈ cycloalkylene group; a C₆-C₁₄ arylene group;
said alkylene, cycloalkylene or arylene groups optionally containing one or more heteroatoms, such as oxygen, nitrogen, sulphur, phosphorus, or one or more carbamoyl or carboxamido groups, and said alkylene, cycloalkylene or arylene groups being optionally substituted by 1 to 5 C₁-C₈ alkyl, C₆-C₁₄ aryl, sulfonate or oxo groups;
- a group selected from divalent groups of the following formulae: —(CH2)ₙ—;—(CH₂),—O—(CH₂)ₘ—O——(CH₂)ₚ—; 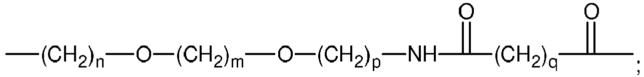 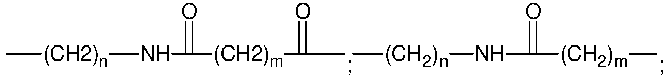 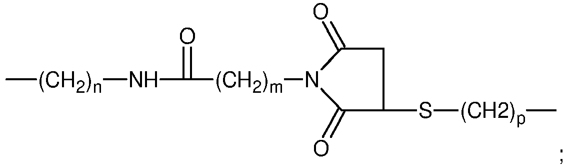 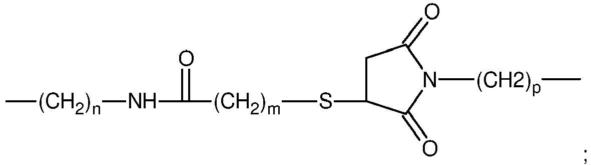 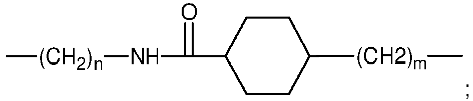 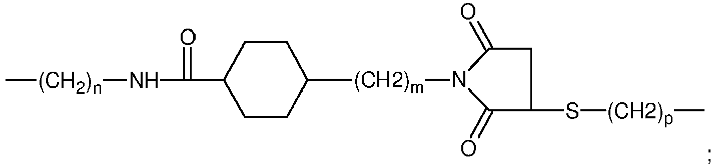 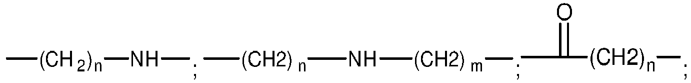 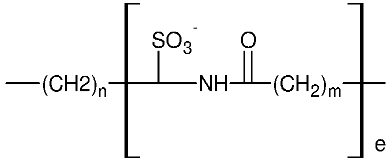 wherein n, m, p, q are integers from 1 to 16, preferably from 1 to 5 and e is an integer from 1 to 6, preferably from 1 to 4.

10. The complexing agent according to any one of the preceding claims, wherein the ―L₂-G group consists of a reactive group G as defined in clam 1, and a spacer arm L₂ consisting of an alkylene chain having from 1 to 5 carbon atoms or a group selected from groups of formula: 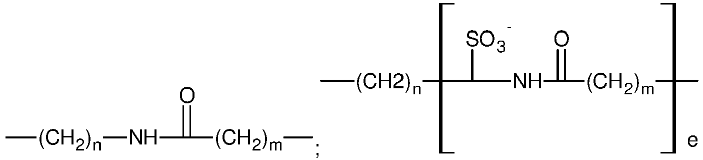 where n, m are integers from 1 to 16, preferably from 1 to 5 and e is an integer from 1 to 6, preferably from 1 to 4, the group G being linked to either end of these divalent groups.

11. A lanthanide complex comprising a complexing agent according to any one of the preceding claims and a lanthanide.

12. The lanthanide complex according to claim 11, **characterized in that** the lanthanide is selected from: Eu³⁺, Sm³⁺, Tb³⁺, Gd³⁺, Dy³⁺, Nd³⁺, Er³⁺.

13. The lanthanide complex according to claim 12, **characterized in that** the lanthanide is Eu³⁺ or Tb³⁺, preferably Eu³⁺.

14. A conjugate obtained by reacting (i) a lanthanide complex according to any one of claims 11 to 13 comprising a group G and (ii) a molecule of interest comprising a functional group, said functional group forming a covalent bond with one of the atoms of group G, said molecule of interest being selected from an amino acid, a peptide, a protein, an antibody, a sugar, a carbohydrate chain, a nucleoside, a nucleotide, an oligonucleotide, and an enzyme substrate.

15. A conjugate according to claim 14, wherein the enzyme substrate is benzylguanine, benzylcytosine, a chloroalkane, or coenzyme A.
